# EUROPEAN PATENT APPLICATION

(11) **EP 4 338 591 A1**
(43) Date of publication of application: **20.03.2024**
(21) Application number: 22807887.9
(22) Date of filing: 13.05.2022
(51) Int. Cl.: A01N 1/02, C12N 1/02, C12N 5/0783

(54) **COMPOSITION FOR NK CELL CRYOPRESERVATION, AND CRYOPRESERVATION FORMULATION COMPRISING SAME**

(30) Priority: 14.05.2021 KR 20210062683
(71) Applicant: GI Cell, Inc., Seongnam-si, Gyeonggi-do 13201 (KR)
(72) Inventor: JANG, Myung Ho, Seoul 05849 (KR); HONG, Chun-Pyo, Seongnam-si Gyeonggi-do 13643 (KR); KO, Dongwoo, Seoul 06601 (KR); KU, Jongbeom, Hanam-si Gyeonggi-do 13012 (KR); KIM, Dong-Hwan, Hanam-si Gyeonggi-do 12919 (KR)
(74) Representative: Mewburn Ellis LLP
(86) International application number: PCT/KR2022/006901
(87) International publication number: WO 2022/240240

(57) **Abstract**

Disclosed are a composition for NK (natural killer) cell cryopreservation and an NK cell cryopreservation formulation. This composition can exhibit cell surface marker expression and NK cell degranulation ability at at least the same level as formulations using commercially available cryopreservation media, even in low-concentration DMSO (dimethyl sulfoxide) not including HEPES (hydroxyethyl piperazine ethane sulfonic acid) or HES (hydroxyethyl starch), and can show a high cell yield and vastly superior ability of NK cells to kill cancer cells. Since a conventional cryoprotectant, showing side effects due to nonspecific toxicity, is not included or is comprised at a low concentration and is replaced with a nontoxic and biocompatible cryoprotectant, the composition can be administered to a patient immediately after thawing without additional culture or replacement of the solution with an injectable solution, and is thus usable for a pharmaceutical formulation. Therefore, the composition is very useful for long-term storage and clinical application of NK-cell-based immune cell therapeutic agents for therapy for very limited indications.

## Description

### BACKGROUND OF THE INVENTION

### Field of the Invention

The present invention relates to a composition for NK (natural killer) cell cryopreservation and an NK cell cryopreservation formulation, and more particularly to a composition for NK cell cryopreservation including or preferably consisting of a base solution, albumin, DMSO (dimethyl sulfoxide), and at least one cryoprotectant selected from the group consisting of glucose, polyethylene glycol, and trehalose, and an NK cell cryopreservation formulation and a pharmaceutical composition including the same.

### Description of the Related Art

Immunotherapy is a method of treating a disease through activation or suppression of the immune system. Since immunotherapy uses bio-derived materials (cytokines, chemokines, interleukins, immune effector cells, etc.), it is biocompatible and thus has fewer side effects and is less likely to cause tolerance issues than conventional drugs (Expert Opinion on Biological Therapy. 1 (4): 641-53). In particular, immune cell therapy, which modulates the immune system by treating immune effector cells such as lymphocytes, macrophages, dendritic cells, natural killer cells (NK cells), and cytotoxic T lymphocytes (CTLs), has recently been gaining more importance, because it has relatively few side effects compared to conventional therapies such as chemotherapy and radiation therapy and shows superior effects when combined with other therapies.

Immune cell therapy strengthens the function of immune cells extracted from a patient or donor, such as T cells, NK cells, dendritic cells, and the like, in an engineered way, amplifies the number of cells through culture, and treats the patient again with such cells, thereby generating an immune response specific to cancer cells to thus induce the death of cancer cells. As such, since a process of culturing immune cells *ex vivo* and then injecting the cells back into the patient is performed, it is also called `adoptive cell transfer' or `adoptive immunotherapy' (CANCER RESEARCH INSTITUTE, 2020-07-09, Adoptive Cell Therapy TIL, TCR, CAR T, AND NK CELL THERAPIES).

In immune cell therapy, research into T-cell-based immune cell therapeutic agents has been most actively conducted to date. In particular, CAR-T cells engineered to express a chimeric antigen receptor (CAR) are advantageous in that cancer-cell-specific attack is possible even when an antigen is not presented on the cell surface through MHC. Currently, various CAR-T cell therapeutic agents such as Kymriah^{®}, Yescarta^{®}, Provenge^{®} and the like have been approved by the FDA and are being used in clinical practice. However, all CAR-T therapeutic agents developed to date target a protein called CD19, which is expressed on B cells, and cause side effects such as B-cell aplasia, cytokine release syndrome (CRS), and neurotoxicity. Hence, clinical application thereof has been limited to acute lymphocytic leukemia and large B-cell lymphoma.

NK-cell-based immune cell therapeutic agents are capable of targeting tumors without antigen pre-sensitization or human leukocyte antigen (HLA) matching. In the case of allogeneic transplantation, these agents rarely induce graft-versus-host disease (GvHD), and unlike T cells, they are less likely to cause cytokine release syndrome due to secretion of IL-3 and granulocyte-macrophage colony-stimulating factors. In addition, the level of PD-1 secreted by NK cells is very low, indicating a high effect on solid tumors. As described above, NK-cell-based immune cell therapeutic agents exhibit many advantages compared to T-cell-based immune cell therapeutic agents, and thus are emerging as a new immune cell therapeutic agent having broad clinical application potential (Cancer Letters 472 (2020): 175-180; Frontiers in Immunology 10 (2019): 2683).

Meanwhile, since the effect of immune cell therapeutic agents is determined depending on the activity of living immune cells, development of a method of storing cells for a long time while maintaining the viability and activity thereof is regarded as important for clinical application. Cryopreservation is the most commonly used method for preserving biological components such as organisms, organs, tissues, cells, and the like (Bioversity International, CATIE, IRD. p.61). However, since cryopreservation may have fatal effects on cells, such as solution effects, intracellular/extracellular ice crystal formation, and dehydration, a freezing method and a cryopreservation formulation for effective preservation of cells must necessarily be developed.

With regard to the freezing method, a slow freezing method such as the slow programmable freezing (SPF) method developed in the 1970s is being used worldwide (Fertility and Sterility. 93 (6): 1921-8). Moreover, for cryopreservation of immune cell therapeutic agents, in order to prevent intracellular/extracellular ice crystal formation, a method of coating cells with a cryoprotectant such as DMSO (dimethyl sulfoxide), glycerol, or HES (hydroxyethyl starch) is used.

However, these cryoprotectants mostly show inherent toxicity and are concentrated in cells during the freezing process, so many studies have reported that they are fatal to immune cell therapeutic agents or that they may be toxic when administered to patients (Peer J. 3: e1039). In particular, it is reported that many cryoprotectants such as DMSO (dimethyl sulfoxide) and HES (hydroxyethyl starch) show high toxicity to human cells (Transfusion and Apheresis Science 46 (2012) 137-147). Thus, cryopreservation formulations including cryoprotectants add additional processing and associated costs to the cell production and preservation process. Moreover, the use of the cryoprotectant alone causes the loss of cell activity and viability, which is undesirable. Therefore, it is required to develop a cryopreservation method and formulation capable of maintaining the activity of immune cell therapeutic agents upon reconstitution by minimizing the concentration of a toxic additive in the composition of a solution for cryopreservation of immune cell therapeutic agents.

Since the expression molecule, signal transduction system, production material, etc. are completely different depending on the types and functions of immune cell therapeutic agents, it is necessary to research and develop customized cryopreservation methods and formulations depending on the types of immune cell therapeutic agents. However, until recently, research on and investment in NK-cell-based immune cell therapeutic agents for clinical application has not been active compared to T-cell-based immune cell therapeutic agents due to difficulties in culture and differentiation thereof, so reports on cryopreservation methods and formulations are lacking.

In order to respond thereto, PCT-US2020-050742 discloses a cryopreservation formulation for an NK cell therapeutic agent. PCT-US2020-050742 discloses an NK cell cryopreservation medium including Plasma-Lyte A, HSA, and DMSO, but is characterized in that DMSO is comprised at a high concentration of 10%. However, in a number of documents such as A. Stolzing et al. (Transfusion and Apheresis Science 46 (2012) 137-147) and Rowley S.D. et al. (Bone Marrow Transplant 2003;31(11) :1043-51), it has been reported that the case in which cryopreserved cells including 10% or more of DMSO are injected into the human body shows toxicity, but that the addition of DMSO at a low concentration is capable of decreasing toxicity and further improving the effect of immune cell therapeutic agents. In addition, an immune cell therapeutic agent formulation including 10% or more of DMSO is reported to show high toxicity, ranging from relatively mild side effects such as nausea, vomiting, chills, and cardiovascular disease (Anticancer Res 1998;18(6B) :4705-8; Bone Marrow Transplant 2000;25(2) :173-7; Transfusion 1991;31(2) :138-41) to serious side effects such as neurotoxicity and nerve edema (Cytotherapy 1999;1(6) :439-46; Neurology 2007;68(11) :859-61.).

Against this background, the present inventors have made great efforts to develop a novel cryopreservation formulation for an NK cell therapeutic agent, developed a composition for NK cell cryopreservation in which a toxic cryoprotectant is used at a low concentration and is replaced with a biocompatible material and a formulation including the same, and ascertained that cryopreservation of the NK cell therapeutic agent with the formulation may result in high cell yield/viability and superior ability to kill cancer cells, thus culminating in the present invention.

The information described in this background section is only for improving understanding of the background of the present invention, and is not to be construed as including information forming the related art already known to those of ordinary skill in the art to which the present invention belongs.

### SUMMARY OF THE INVENTION

It is an object of the present invention to provide a composition for NK cell cryopreservation for effectively cryopreserving NK cells.

It is another object of the present invention to provide an NK cell cryopreservation formulation.

It is still another object of the present invention to provide a method of cryopreserving NK cells using the composition for NK cell cryopreservation.

It is yet another object of the present invention to provide a cell therapeutic agent including NK cells and the composition for NK cell cryopreservation.

It is still yet another object of the present invention to provide the use of the NK cells and the composition for NK cell cryopreservation for the prevention or treatment of cancer, an immune disease, or an infectious disease.

In order to accomplish the above objects, the present invention provides a composition for NK cell cryopreservation including (i) a base solution, (ii) serum albumin, (iii) DMSO, and (iv) at least one cryoprotectant selected from the group consisting of glucose, polyethylene glycol, and trehalose.

In addition, the present invention provides the use of the composition for NK cell cryopreservation for the manufacture of an NK cell cryopreservation formulation.

In addition, the present invention provides an NK cell cryopreservation formulation including NK cells and the composition for NK cell cryopreservation.

In addition, the present invention provides a method of cryopreserving NK cells including (a) preparing an NK cell cryopreservation formulation by suspending NK cells in the composition for NK cell cryopreservation and (b) freezing the NK cell cryopreservation formulation.

In addition, the present invention provides an immune cell therapeutic agent including NK cells and the composition for NK cell cryopreservation.

In addition, the present invention provides a pharmaceutical composition for preventing or treating cancer, an immune disease, or an infectious disease including NK cells and the composition for NK cell cryopreservation.

In addition, the present invention provides a method of preventing or treating cancer, an immune disease, or an infectious disease using the NK cell cryopreservation formulation, the immune cell therapeutic agent, or the pharmaceutical composition.

In addition, the present invention provides the use of the NK cell cryopreservation formulation or the immune cell therapeutic agent for the manufacture of a pharmaceutical composition for the prevention or treatment of cancer, an immune disease, or an infectious disease.

In addition, the present invention provides the use of the NK cell cryopreservation formulation, the immune cell therapeutic agent, or the pharmaceutical composition for the prevention or treatment of cancer, an immune disease, or an infectious disease.

### BRIEF DESCRIPTION OF THE DRAWINGS

The above and other objects, features, and other advantages of the present invention will be more clearly understood from the following detailed description taken in conjunction with the accompanying drawings, in which:
FIGS. 1A and 1B show the results of measurement of cell yield (FIG. 1A) and viability (FIG. 1B) after cryopreserving NK cell cryopreservation formulations for 4 weeks and then thawing the same, in which the number of trials for each group was as follows: Comparative Example (n=12), candidate 1-1 (n=11), candidate 1-2 (n=2), candidate 1-3 (n=2), candidate 2-1 (n=10), candidate 2-2 (n=2), candidate 3-1 (n=9), and candidate 3-2 (n=2);
FIG. 2 shows results confirming surface markers indicating the activity or inhibition of NK cells after thawing NK cells cryopreserved in individual formulations, in which the number of trials for each group was as follows: Comparative Example (n=20), candidate 1-1 (n=16), candidate 1-2 (n=6), candidate 1-3 (n=3), candidate 2-1 (n=17), candidate 2-2 (n=3), candidate 3-1 (n=17), and candidate 3-2 (n=3);
FIG. 3 shows results confirming cytotoxicity markers (degranulation markers) of NK cells after thawing NK cells cryopreserved in individual formulations, in which the number of trials for each group was as follows: Comparative Example (n=20), candidate 1-1 (n=16), candidate 1-2 (n=6), candidate 1-3 (n=3), candidate 2-1 (n=17), candidate 2-2 (n=3), candidate 3-1 (n=17), and candidate 3-2 (n=3);
FIGS. 4A to 4D show results confirming the ability of NK cells to kill target cancer cells (K562) at each E:T ratio after thawing NK cells cryopreserved in individual formulations;
FIGS. 5A to 5D show results confirming the ability of NK cells to kill target cancer cells (BT474) at each E:T ratio after thawing NK cells cryopreserved in individual formulations;
FIGS. 6A to 6D show results confirming the ability of NK cells to kill target cancer cells (MDA-MB-231) at each E:T ratio after thawing NK cells cryopreserved in individual formulations;
FIGS. 7A and 7B show results confirming the yield and viability of NK cells after thawing NK cells cryopreserved in formulations including PEG at various average molecular weights and concentrations;
FIG. 8 shows results confirming cell surface markers of NK cells after thawing NK cells cryopreserved in formulations including PEG at various average molecular weights and concentrations;
FIG. 9 shows results confirming cytotoxicity markers of NK cells after thawing NK cells cryopreserved in formulations including PEG at various average molecular weights and concentrations; and
FIGS. 10A and 10B show results confirming the ability of NK cells to kill cancer cells after thawing NK cells cryopreserved in formulations including PEG at various average molecular weights and concentrations, FIG. 10A showing the ability of thawed NK cells to kill K562 cancer cells and FIG. 10B showing the ability of thawed NK cells to kill DLD-1 cancer cells.

### DETAILED DESCRIPTION OF THE INVENTION

Unless otherwise defined, all technical and scientific terms used herein have the same meanings as those typically understood by those skilled in the art to which the present invention belongs. In general, the nomenclature used herein is well known in the art and is typical.

In the concentration range described herein, "lower limit value to upper limit value" means that lower and upper limit values are included (`lower limit value or more' and 'upper limit value or less'), and when these limit values are not included, the concentration range is described as "more than" or "less than". As used for numerical values herein, the term "about" is used to include a range expected to have an effect substantially equivalent to a numerical value provided by those skilled in the art, and may be, for example, ±20%, ±10%, ±5%, etc. of the provided numerical value, but the present invention is not limited thereto.

NK-cell-based immune cell therapeutic agents exhibit many advantages compared to T-cell-based immune cell therapeutic agents, and have thus recently emerged as a new immune cell therapeutic agent having broad clinical application potential (Cancer Letters 472 (2020): 175-180; Frontiers in Immunology 10 (2019): 2683).

Since the effect of immune cell therapeutic agents is determined depending on the activity of living immune cells, development of a method of storing cells for a long time while maintaining the viability and activity thereof is regarded as important for clinical application. For cryopreservation, which is a general storage method, optimization of the cryopreservation formulation must also be achieved in order to overcome limitations such as solution effects, intracellular/extracellular ice crystal formation, and dehydration.

As conventional cryoprotectants against intracellular/extracellular ice crystal formation, etc., DMSO (methyl sulfoxide), HEPES (hydroxyethyl piperazine ethane sulfonic acid), and HES (hydroxyethyl starch) have been commonly used at high concentrations (about 8-10% (v/v) or more). However, such a cryoprotectant material shows inherent toxicity and is concentrated in cells during the freezing process, so many studies have reported that it is fatal to immune cell therapeutic agents or is toxic when administered to a patient (Peer J. 3: e1039).

In an embodiment of the present invention, a composition for NK cell cryopreservation, which includes DMSO at a low concentration, without including HEPES (hydroxyethyl piperazine ethane sulfonic acid) or HES (hydroxyethyl starch), and in which a cryoprotectant having inherent nonspecific toxicity is replaced with a biocompatible and nontoxic additive (glucose, PEG, and trehalose), was designed.

In another embodiment of the present invention, based on results of comparison of cell yield/viability, cell surface marker expression, and toxic marker expression after addition of a cryoprotectant (glucose, PEG, and/or trehalose) at various component ratios, it was confirmed that cell viability and marker expression characteristics equal to or higher than those of commercially available compositions for cryopreservation were exhibited, despite the use of DMSO at a low concentration without including HEPES (hydroxyethyl piperazine ethane sulfonic acid) or HES (hydroxyethyl starch), and also that cell yield and ability to kill target cancer cells were remarkably increased.

Accordingly, an aspect of the present invention pertains to a composition for NK cell cryopreservation including
(i) a base solution;
(ii) serum albumin;
(iii) DMSO; and
(iv) at least one cryoprotectant selected from the group consisting of glucose, polyethylene glycol, and trehalose.

As used herein, the term "base solution" refers to a solution that has properties similar to those of human blood or body fluid and thus does not damage NK cells, and preferably means a liquid composition that is included in the largest volume proportion in the composition for NK cell cryopreservation according to the present invention to become a base.

In the present invention, the base solution may include at least one selected from the group consisting of sodium, chloride, potassium, magnesium, acetate (and/or lactate), and gluconate.

In the present invention, the base solution may include at least one selected from among sodium, chloride, potassium, magnesium, acetate, and gluconate, and preferably includes sodium, chloride, potassium, magnesium, acetate, and gluconate.

In the present invention, the base solution may include, in addition to the materials disclosed above, additional salts, sugars, lipids, proteins, and the like, so long as they do not negatively affect the activity or viability of NK cells.

In the present invention, the base solution may have an appropriate pH and osmolality in order to prevent damage to NK cells, and preferably has a pH and osmolality similar to those of human plasma or body fluid.

In the present invention, the pH of the base solution may be about 4.0 to about 8.0, preferably about 6.0 to about 8.0, more preferably about 7.0 to about 8.0, most preferably about 7.4.

In the present invention, the osmolality of the base solution may be about 200 mOsm/L to about 400 mOsm/L, preferably about 250 mOsm/L to about 350 mOsm/L, more preferably about 280 mOsm /L to about 310 mOsm/L, most preferably about 300 mOsm/L or about 294 mOsm/L.

In the present invention, the base solution may include a commercially available fluid or a modified composition thereof. For example, Plasma Solution A, Plasma-Lyte (e.g. Plasma-Lyte A, Plasma-Lyte 148, Plasma-Lyte 56, etc.), Hartmann's solution, Ringer's solution, about 0.5% saline, Sterofundin, or solutions having a composition similar thereto may be used, and preferably Plasma Solution A or Plasma-Lyte (e.g. Plasma-Lyte A, Plasma-Lyte 148, Plasma-Lyte 56, etc.) is used.

In an embodiment of the present invention, Plasma Solution A, having the following composition, was used, and the composition of Plasma Solution A included in the composition for NK cell cryopreservation prepared in Example of the present invention was as follows.

| Classification | Composition | Volume fraction in composition for cryopreservation | Final concentration in composition for cryopreservation |
|---|---|---|---|
| Plasma Solution A | Magnesium chloride; | 50% (v/v) | Na:0.1609286% (w/v) |
| | Potassium chloride | | Cl:0.1737197% (w/v) |
| | | | K:0.009774575% (w/v) |
| | Sodium acetate | | Mg:0.0018% (w/v) |
| | | | Acetate:0.0810702% (w/v) |
| | Sodium chloride | | |
| | | | Gluconate: 0.225584% (w/v) |
| | Sodium gluconate | | |

In the present invention, the base solution may include sodium, chloride, potassium, magnesium, acetate, and gluconate, like Plasma Solution A.

In the present invention, the final concentration of each component included in Plasma Solution A in the composition for cryopreservation may vary depending on the fraction (% (v/v)) of the base solution included in the composition for cryopreservation.

Accordingly, the final concentration (% (w/v)) of sodium, chloride, potassium, magnesium, acetate, and gluconate included in the base solution of the composition for NK cell cryopreservation according to the present invention preferably corresponds to ±20%, more preferably ±10% of the final concentration of the above table in Example. Even in this case, it will be apparent to those skilled in the art that effects substantially equivalent to those confirmed in an embodiment of the present invention can be expected.

In the present invention, the base solution may be the remaining component fraction except for the preferred fractions of other components to be described below.

In the present invention, the composition for NK cell cryopreservation may include albumin.

As used herein, the term "albumin" refers to the largest protein component of human blood (about 50-60%), and is involved in plasma osmotic pressure, intracellular delivery of nutrients and ions, immunity, and energy metabolism.

In the present invention, the albumin may be serum albumin, preferably human serum albumin.

According to the present invention, the albumin may be comprised at a final concentration of about 1% (w/v) to about 10% (w/v), preferably about 2% (w/v) to about 8% (w/v), more preferably about 3% (w/v) to about 5% (w/v), most preferably about 4% (w/v).

In the present invention, the albumin may be replaced with or included in combination with another protein that performs a function similar thereto. Examples of protein performing a function similar to albumin may include, but are not limited to, alpha-fetoprotein, vitamin-D-binding protein, and the like.

In the present invention, the composition may include DMSO.

As used herein, the term "DMSO (dimethyl sulfoxide)" is a compound obtained by oxidizing dimethyl sulfide, and is the most frequently used cryoprotectant for protecting cells from intracellular/external ice crystal formation, etc. upon cryopreservation of cells. However, in various documents, DMSO is reported to cause various kinds of cell damage due to nonspecific cytotoxicity (Transfusion and Apheresis Science 46 (2012) 137-147; and Bone Marrow Transplant 2003;31(11):1043-51). The cell damage mechanism by DMSO has been reported to be due to a rapid increase in intracellular osmotic pressure, mitochondrial damage, etc. In particular, various side effects have been reported, such as causing abnormal implantation of a fertilized egg in women of childbearing age and causing serious damage to the development of the fetus during pregnancy (Theranostics (IF: 8.712)). Also, HEPES and HES used as cryoprotectants, in addition to DMSO, have been reported to show nonspecific cytotoxicity (Transfusion and Apheresis Science 46 (2012) 137-147). It has been reported that about 10% (v/v) DMSO is fatal to cells but that about 5% (v/v) DMSO or less is not fatal to cells.

Accordingly, the composition for NK cell cryopreservation according to the present invention may include DMSO at a low concentration.

In the present invention, the DMSO may be comprised at a final concentration of about 0.001% (v/v) to less than about 10% (v/v), preferably about 0.005% (v/v) to about 8% (v/v), more preferably about 0.1% (v/v) to about 6% (v/v), and most preferably about 5% (v/v).

In the present invention, the composition for NK cell cryopreservation does not include HEPES or HES.

As in conventionally reported and commercially available compositions for cell cryopreservation, the concentration of DMSO that is generally used for a sufficient cryoprotective effect is about 8-10% (v/v) or more. The medium composition of the present invention uses low-concentration DMSO and thus does not show significant side effects or toxicity. Therefore, an additional cryoprotectant may be included for a sufficient cryoprotective effect.

In the present invention, at least one cryoprotectant selected from the group consisting of glucose, polyethylene glycol, and trehalose is used.

In the present invention, the cryoprotectant may be any one selected from the group consisting of glucose, polyethylene glycol, and trehalose.

In the present invention, the polyethylene glycol may have an average molecular weight of about 100 g/mol to about 20000 g/mol, preferably about 200 g/mol to about 20000 g/mol, more preferably about 400 g/mol to about 20000 g/mol, most preferably about 200 g/mol, about 400 g/mol, about 2000 g/mol, or about 20000 g/mol.

In the present invention, the average molecular weight is the average of molecular weights of molecules of polyethylene glycol included in the cryopreservation formulation.

In the present invention, the polyethylene glycol included in the cryopreservation formulation may have a molecular weight corresponding to about ±50%, preferably about ±40%, more preferably about ±30%, much more preferably about ±20%, most preferably about ±10% or about ±5% of the average molecular weight described above.

In the present invention, when the cryoprotectant is glucose, it may be comprised at a final concentration of about 0.5% (w/v) to about 5% (w/v), preferably about 1% (w/v) to about 4% (w/v), more preferably about 1% (w/v) to about 3% (w/v), most preferably about 1.25% (w/v) to about 2.5% (w/v).

In the present invention, when the cryoprotectant is polyethylene glycol, it may be comprised at a final concentration of about 0.01% (w/v) to about 10% (w/v), preferably about 0.02% (w/v) to about 6% (w/v), more preferably about 0.04% (w/v) to about 3% (w/v), most preferably about 0.045% (w/v) to about 2.5% (w/v), about 0.225% (w/v) to about 2.5% (w/v), about 0.625% (w/v) to about 2.5% (w/v), about 0.045% (w/v) to about 2.25% (w/v), about 0.225% (w/v) to about 2.25% (w/v), or about 0.625% (w/v) to about 2.25% (w/v).

In the present invention, when the polyethylene glycol has an average molecular weight of about 400 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.625% (w/v) to about 2.5% (w/v).

In the present invention, when the polyethylene glycol has an average molecular weight of about 4000 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.225% (w/v) to about 2.25% (w/v).

In the present invention, when the polyethylene glycol has an average molecular weight of about 20000 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.045% (w/v) to about 2.25% (w/v).

In the present invention, when the cryoprotectant is trehalose, it may be comprised at a final concentration of about 0.5% (w/v) to about 10% (w/v), preferably about 1% (w/v) to about 9% (w/v), more preferably about 1.7% (w/v) to about 8.5% (w/v).

According to the present invention, the human serum albumin may be comprised at a final concentration of about 1% (w/v) to about 10% (w/v), preferably about 2% (w/v) to about 8% (w/v), more preferably about 3% (w/v) to about 5% (w/v), most preferably about 4% (w/v).

In the present invention, the composition for NK cell cryopreservation preferably consists of
(i) a base solution consisting of sodium, chloride, potassium, magnesium, acetate, and gluconate;
(ii) human serum albumin;
(iii) DMSO; and
(iv) at least one cryoprotectant selected from the group consisting of glucose, polyethylene glycol, and trehalose.

In the present invention, the base solution may have an appropriate pH and osmolality in order to prevent damage to NK cells, and preferably has a pH and osmolality similar to those of human plasma or body fluid.

In the present invention, the pH of the base solution may be about 4.0 to about 8.0, preferably about 6.0 to about 8.0, more preferably about 7.0 to about 8.0, most preferably about 7.4.

In the present invention, the osmolality of the base solution may be about 200 mOsm/L to about 400 mOsm/L, preferably about 250 mOsm/L to about 350 mOsm/L, more preferably about 280 mOsm/L to about 310 mOsm/L, most preferably about 300 mOsm/L or about 294 mOsm/L.

In the present invention, the base solution may include a commercially available fluid or a modified composition thereof. Examples thereof may include, but are not limited to, Plasma Solution A, Plasma-Lyte (e.g. Plasma-Lyte A, Plasma-Lyte 148, Plasma-Lyte 56, etc.), and solutions having a composition similar thereto.

In the present invention, it is apparent to those skilled in the art that the final concentration of each component included in Plasma Solution A in the composition for cryopreservation is determined depending on the fraction (% (v/v)) of the base solution included in the composition for cryopreservation. Therefore, the final concentration (% (w/v)) of each of sodium, chloride, potassium, magnesium, acetate, and gluconate constituting the base solution of the composition for NK cell cryopreservation according to the present invention is preferably ±20%, more preferably ±10% of the final concentration of each component described in Example. Even in this case, effects substantially equivalent to those confirmed in an embodiment of the present invention can be expected.

In the present invention, the human serum albumin may be comprised at a final concentration of about 1% (w/v) to about 10% (w/v), preferably about 2% (w/v) to about 8% (w/v), more preferably about 3% (w/v) to about 5% (w/v), most preferably about 4% (w/v).

In the present invention, the DMSO may be comprised at a final concentration of about 0.001% (v/v) to less than about 10% (v/v), preferably about 0.005% (v/v) to about 8% (v/v), more preferably about 0.1% (v/v) to about 6% (v/v), most preferably about 5% (v/v).

In the present invention, the cryoprotectant may be any one selected from the group consisting of glucose, polyethylene glycol, and trehalose.

In the present invention, after thawing cryopreservation formulations using, as the cryoprotectant, polyethylene glycol having various average molecular weights, for example, PEG-400, PEG-4000, or PEG-20000 at various concentrations, the yield and viability of NK cells were confirmed.

In the present invention, the polyethylene glycol may have an average molecular weight of about 100 g/mol to about 20000 g/mol, preferably about 200 g/mol to about 20000 g/mol, more preferably about 400 g/mol to about 20000 g/mol, most preferably about 200 g/mol, about 400 g/mol, about 2000 g/mol, or about 20000 g/mol.

In the present invention, the average molecular weight is the average of molecular weights of molecules of polyethylene glycol included in the cryopreservation formulation.

In the present invention, the polyethylene glycol included in the cryopreservation formulation may have a molecular weight corresponding to about ±50%, preferably about ±40%, more preferably about ±30%, much more preferably about ±20%, most preferably about ±10% or about ±5% of the average molecular weight described above.

In the present invention, when the cryoprotectant is glucose, it may be comprised at a final concentration of about 0.5% (w/v) to about 5% (w/v), preferably about 1% (w/v) to about 4% (w/v), more preferably about 1% (w/v) to about 3% (w/v), most preferably about 1.25% (w/v) to about 2.5% (w/v).

In the present invention, when the cryoprotectant is polyethylene glycol, it may be comprised at a final concentration of about 0.01% (w/v) to about 10% (w/v), preferably about 0.02% (w/v) to about 6% (w/v), more preferably about 0.04% (w/v) to about 3% (w/v), most preferably about 0.045% (w/v) to about 2.5% (w/v), about 0.225% (w/v) to about 2.5% (w/v), about 0.625% (w/v) to about 2.5% (w/v), about 0.045% (w/v) to about 2.25% (w/v), about 0.225% (w/v) to about 2.25% (w/v), or about 0.625% (w/v) to about 2.25% (w/v).

In the present invention, when the polyethylene glycol has an average molecular weight of about 400 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.625% (w/v) to about 2.5% (w/v).

In the present invention, when the polyethylene glycol has an average molecular weight of about 4000 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.225% (w/v) to about 2.25% (w/v).

In the present invention, when the polyethylene glycol has an average molecular weight of about 20000 g/mol, it may be comprised at a final concentration of about 0.01% (w/v) to about 10% (w/v), preferably about 0.02% (w/v) to about 6% (w/v), more preferably about 0.045% (w/v) to about 2.5% (w/v), most preferably about 0.045% (w/v) to about 2.25% (w/v).

In the present invention, when the cryoprotectant is trehalose, it may be comprised at a final concentration of about 0.5% (w/v) to about 10% (w/v), preferably about 1% (w/v) to about 9% (w/v), more preferably about 1.7% (w/v) to about 8.5% (w/v).

In the present invention, the human serum albumin may be comprised at a final concentration of about 1% (w/v) to about 10% (w/v), preferably about 2% (w/v) to about 8% (w/v), more preferably about 3% (w/v) to about 5% (w/v), most preferably about 4% (w/v).

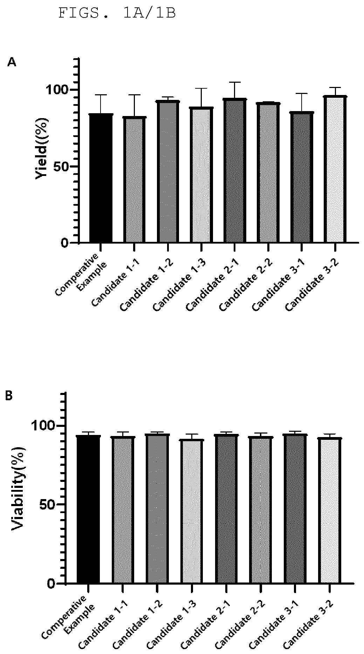

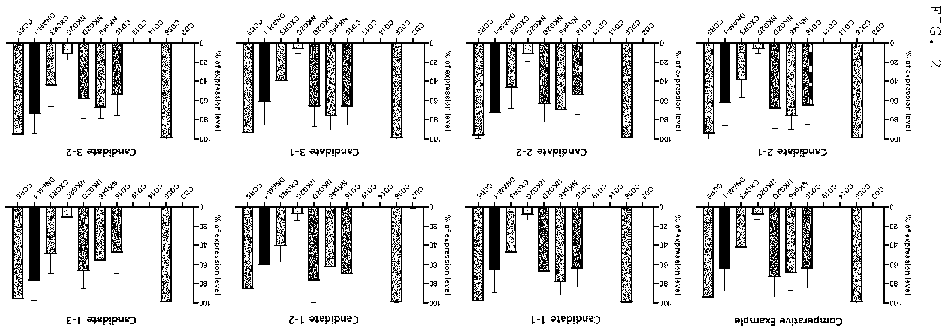

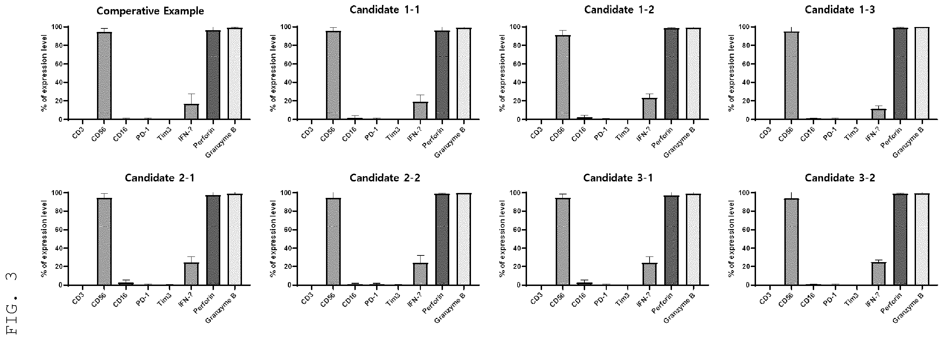

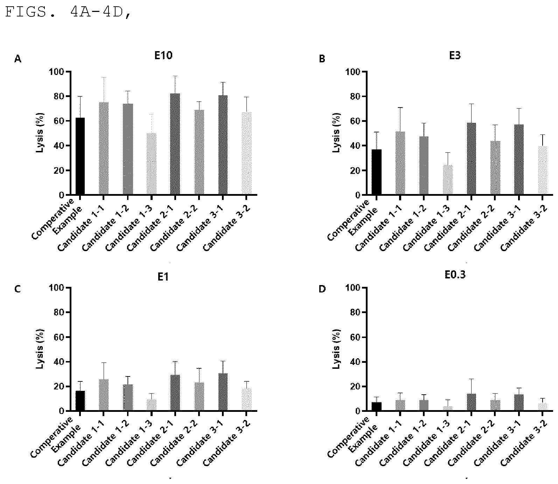

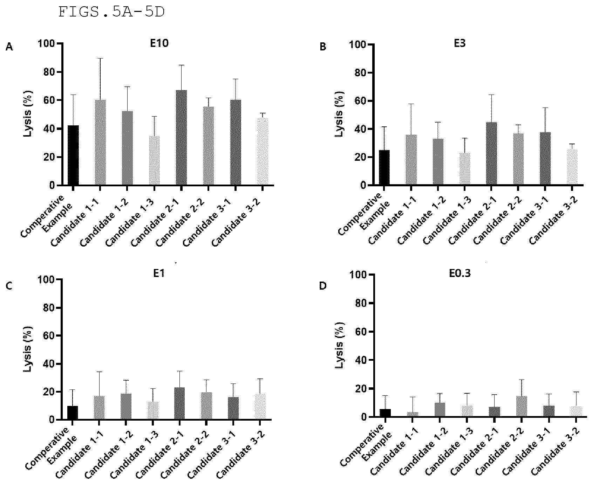

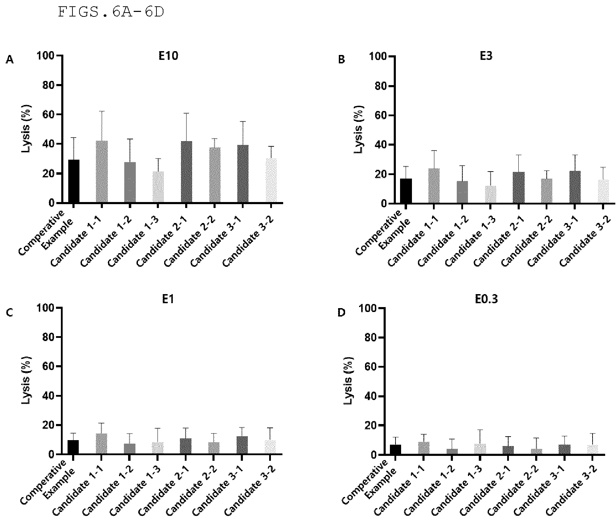

As confirmed in an embodiment of the present invention, candidates 1-1 and 1-2, candidates 2-1 and 2-2, and candidate 3-1 (Table 1) in Examples of the present invention showed cell surface marker expression (FIG. 2) and NK cell degranulation ability (FIG. 3) at the same level as the formulation using the conventional cryopreservation medium (CS10), even in low-concentration DMSO not including HEPES or HES, and also showed a high cell yield (FIG. 1A) and vastly superior ability of NK cells to kill cancer cells in practice (FIGS. 4A-4D to 6A-6D). Among them, candidate 2-1 showed the highest yield when thawed, and stably exhibited high ability to kill cancer cells.

Another aspect of the present invention pertains to an NK cell cryopreservation formulation including NK cells and the composition for NK cell cryopreservation of the present invention.

In the present invention, the NK cell cryopreservation formulation may include additional components such as energy sources and other proteins in addition to the NK cells and the composition for NK cell cryopreservation of the present invention.

As used herein, the term "NK cell" is called a `natural killer cell' and induces MHC-independent cell death as a constituent of innate immunity. In the present invention, the term "NK cells" is used in a broad sense encompassing not only NK cells naturally present in living organisms, but also genetically engineered NK cells (e.g. CAR-NK, etc.) and NK-cell-based cell therapeutic agents. The NK cells may be identified by various surface markers. For example, the NK cells may be CD3(-)CD56(+) NK cells, but are not limited thereto.

In this aspect, the composition for NK cell cryopreservation may share the same characteristics as described above.

Still another aspect of the present invention pertains to a method of cryopreserving NK cells including (a) preparing an NK cell cryopreservation formulation by suspending NK cells in the composition for NK cell cryopreservation and (b) freezing the NK cell cryopreservation formulation.

In the present invention, in step (a), a cryopreservation formulation may be prepared by suspending NK cells at a concentration of about 1×10² cells/mL to about 1×10¹³ cells/mL, preferably about 1×10⁵ cells/mL to about 1×10⁹ cells/mL.

In the present invention, the `freezing' in step (b) may be performed through a typical method. For example, freezing may be performed through a method such as vitrification, slow freezing, or the like, but is not limited thereto. In the freezing process, the composition for NK cell cryopreservation of the present invention serves to prevent the formation of intracellular ice crystals and protect cells, and to significantly increase the yield/viability of cells after thawing.

As used herein, the term "vitrification" means that cells are cooled and thus vitrified (Nature. 313 (6003): 573-5; Cryo Letters. 25 (4): 241-54). Vitrification is advantageous in that cryopreservation is possible without the formation of ice crystals. Vitrification may be carried out by relatively rapidly decreasing the temperature, such as cooling to the final temperature in ones of minutes to ones of hours. Vitrification may be performed through a typical method, and the final temperature and the temperature decrease rate may be calculated through methods well known in the art depending on the type of cell, cryopreservation formulation, and the like (G. Allahbadia et al., Vitrification in Assisted Reproduction, © Springer India 2015). For example, the vitrification process may be performed in a manner in which a cryopreservation formulation is prepared by suspending cells in the composition for NK cell cryopreservation of the present invention, the temperature is lowered within ones of minutes to ones of hours using equipment such as controlled rate freezing (CRF), and the frozen cells are stored in a nitrogen tank when the final temperature is reached, but the present invention is not limited thereto.

As used herein, "slow freezing" or "slow cooling" refers to a process of slowly freezing cells by lowering the temperature relatively slowly, and is interchangeable with "slow programmable freezing". Slow freezing may be carried out by relatively slowly decreasing the temperature, such as cooling to the final temperature in ones of hours to tens of hours. Slow freezing may be performed through a typical method, and the final temperature and the temperature decrease rate may be calculated through methods well known in the art depending on the type of cell, cryopreservation formulation, and the like. For example, the slow freezing process may be performed in a manner in which a cryopreservation formulation is prepared by suspending cells in the composition for NK cell cryopreservation of the present invention, a vial containing the cryopreservation formulation is placed in a freezing container box containing isopropyl alcohol, and cells are frozen by lowering the temperature to a predetermined level in ones to tens of hours in a cryogenic freezer, but the present invention is not limited thereto.

In the present invention, cryoprotectants such as HES and/or HEPES, which are toxic and cause side effects in the human body, are not included, and low-concentration DMSO, at a level that is not harmful to the human body, is included as a cryoprotectant. The combination of a biocompatible and nontoxic cryoprotectant makes it possible to exhibit a marked improvement in the ability to kill target cancer cells, as well as high viability/yield of NK cells. Due to minimization of the toxic cryoprotectant, the formulation including the NK cells and the composition for NK cell cryopreservation of the present invention may be administered to a subject without additional processing or treatment to thus exhibit the intended effect of the NK cell therapeutic agent in the subject.

Accordingly, yet another aspect of the present invention pertains to an immune cell therapeutic agent including NK cells and the composition for NK cell cryopreservation of the present invention.

As used herein, the term "immune cell therapeutic agent" refers to an agent used for immunotherapy that directly/indirectly activates the immune system of the administered subject or treats a disease by directly affecting cells, and indicates a therapeutic agent including immune cells extracted from a patient or an allogeneic or xenogeneic subject or engineered immune cells.

In the present invention, the immune cell therapeutic agent may be used after thawing the cryopreserved NK cell cryopreservation formulation of the present invention.

As used herein, the term "thawing" refers to thawing and reactivation of cryopreserved cells. In the present invention, thawing may be performed through a typical method known in the art. For example, it may be carried out by immersing a vessel containing a cryopreserved immune cell therapeutic agent in a water bath preheated to about 37°C for about 1 hour or more, but the present invention is not limited thereto, and the temperature and the thawing time may be adjusted depending on the type of cell, cryopreservation formulation, dose, etc.

In particular, the immune cell therapeutic agent of the present invention does not include HEPES (hydroxyethyl piperazine ethane sulfonic acid) or HES (hydroxyethyl starch), which is a cryoprotectant known to be toxic to the living body, and includes DMSO at a much lower concentration than conventional compositions for cryopreservation, and thus may be used immediately without the need to remove a harmful cryoprotectant in a separate step.

In the present invention, the immune cell therapeutic agent may be used immediately after thawing (ready to use) .

As used herein, the term "ready to use" refers to immediate use/administration at a pharmaceutically effective concentration without an additional dilution/concentration, reconstitution, or rehydration process after thawing.

Since the immune cell therapeutic agent of the present invention is in a form that may be used immediately (ready to use), user convenience is improved, and it is also possible to prevent activity variation, contamination, and the like caused by errors that may occur during dilution/concentration, reconstitution, or rehydration.

In the present invention, the immune cell therapeutic agent may include NK cells at a medically or pharmaceutically effective concentration. For example, the immune cell therapeutic agent may include NK cells at a final concentration of 1×10² to 1×10¹³ cells/mL, preferably 1×10⁵ to 1×10⁹ cells/mL.

Still yet another aspect of the present invention pertains to a pharmaceutical composition for preventing or treating cancer, an immune disease, or an infectious disease including NK cells and the composition for NK cell cryopreservation of the present invention.

In the present invention, the pharmaceutical composition may be used or administered after thawing the NK cryopreservation formulation of the present invention.

In the present invention, the pharmaceutical composition may be used immediately after thawing (ready to use).

In the present invention, the pharmaceutical composition may include NK cells at a final concentration of 1×10² to 1×10¹³ cells/mL, preferably 1×10⁵ to 1×10⁹ cells/mL.

In the present invention, the NK cells are used in a broad sense encompassing not only NK cells naturally present in living organisms, but also genetically engineered NK cells (e.g. CAR-NK, etc.) and NK-cell-based cell therapeutic agents. The NK cells may be identified by various surface markers. For example, the NK cells may be CD3(-)CD56(+) NK cells, but are not limited thereto.

Even yet another aspect of the present invention pertains to a method of preventing or treating cancer, an immune disease, or an infectious disease including administering the pharmaceutical composition.

Even still yet another aspect of the present invention pertains to a method of preventing or treating cancer, an immune disease, or an infectious disease using the NK cell cryopreservation formulation, immune cell therapeutic agent, or pharmaceutical composition of the present invention.

In the present invention, the NK cell cryopreservation formulation, immune cell therapeutic agent, or pharmaceutical composition of the present invention may be administered to a subject for the purpose of preventing or treating cancer, an immune disease, or an infectious disease.

In the present invention, the NK cell cryopreservation formulation, immune cell therapeutic agent, or pharmaceutical composition of the present invention may be used immediately after thawing (ready to use) .

A further aspect of the present invention pertains to the use of the NK cell cryopreservation formulation of the present invention or the cryopreservation formulation for the immune cell therapeutic agent or pharmaceutical composition for the manufacture of a pharmaceutical composition for the prevention or treatment of cancer, an immune disease, or an infectious disease.

Still a further aspect of the present invention pertains to the use of the NK cell cryopreservation formulation of the present invention or the cryopreservation formulation for the immune cell therapeutic agent or pharmaceutical composition for the prevention or treatment of cancer, an immune disease, or an infectious disease.

Yet a further aspect of the present invention pertains to the use of the NK cells and the composition for NK cell cryopreservation of the present invention for the prevention or treatment of cancer, an immune disease, or an infectious disease.

Still yet a further aspect of the present invention pertains to the use of the NK cells and the composition for NK cell cryopreservation of the present invention for the manufacture of a pharmaceutical composition for the prevention or treatment of cancer, an immune disease, or an infectious disease.

In the present invention, the term 'cancer' is used in the same sense as 'tumor', and the pharmaceutical composition for prevention or treatment according to the present invention is applicable to all types of cancer including solid cancer and blood cancer. Unlike blood cancer, solid cancer is cancer resulting from formation of a lump in an organ, and cancer occurring in most organs is solid cancer. The type of cancer that may be treated using the immune cell therapeutic agent or pharmaceutical composition according to the present invention is not particularly limited, and examples thereof may include, but are not limited to, gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumors, neuroblastoma, retinoblastoma, head and neck cancer, salivary gland cancer, lymphoma, and the like.

In the present invention, the `immune disease' is a disease caused by an abnormal immune response, and may be exemplified by an autoimmune disease, transplant rejection, or arthritis, but is not limited thereto. Examples of the autoimmune disease may include, but are not limited to, Crohn's disease, erythematosus, atopy, rheumatoid arthritis, Hashimoto's thyroiditis, pernicious anemia, Addison's disease, type 1 diabetes, lupus, chronic fatigue syndrome, fibromyalgia, hypothyroidism and hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma, and ulcerative colitis. The transplant rejection may be allograft rejection or graft-versus-host disease (GVHD).

In the present invention, the `infectious disease' is a disease caused by infection with a virus or pathogen, and is a concept that includes all diseases that may be transmitted and infected through the respiratory system, blood, skin contact, etc. Non-limiting examples of the infectious disease may include, but are not limited to, hepatitis B and C, human papilloma virus (HPV) infection, cytomegalovirus infection, viral respiratory disease, influenza, and the like.

As used herein, the term "prevention" refers to any action that suppresses or delays the progression of cancer, an immune disease, or an infectious disease by administering the pharmaceutical composition for prevention or treatment of the present invention, and "treatment" refers to suppressing the development of or alleviating or eliminating symptoms of cancer, an immune disease, or an infectious disease.

The pharmaceutical composition for prevention or treatment according to the present invention may include a pharmaceutically effective amount of NK cells alone, or may include one or more pharmaceutically acceptable carriers, excipients, or diluents. Here, the pharmaceutically effective amount is an amount sufficient to prevent, ameliorate, and eliminate symptoms of cancer, an immune disease, or an infectious disease.

"Pharmaceutically acceptable" means that a composition is physiologically acceptable and does not usually cause allergic reactions such as gastrointestinal disorders and dizziness or reactions similar thereto when administered to humans. Examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. In addition, fillers, anti-agglomeration agents, lubricants, wetting agents, fragrances, emulsifiers, and preservatives may be further included.

In addition, the pharmaceutical composition for prevention or treatment of the present invention may include at least one known active ingredient having a therapeutic effect on cancer, an immune disease, or an infectious disease, in addition to the NK cells and the composition for NK cell cryopreservation of the present invention.

The pharmaceutical composition for prevention or treatment of the present invention may be formulated using any method known in the art in order to provide rapid, sustained, or delayed release of the active ingredient after administration. Formulations may be in the form of powders, granules, tablets, emulsions, syrups, aerosols, soft or hard gelatin capsules, sterile injectable solutions, or sterile powders.

The pharmaceutical composition for prevention or treatment of the present invention may be administered through various routes including oral, transdermal, subcutaneous, intravenous, or intramuscular routes, and the dosage of the active ingredient may be appropriately determined depending on various factors including the route of administration, age, gender, and body weight of the patient, severity of disease, and the like. The pharmaceutical composition for prevention or treatment according to the present invention may be administered in combination with a known compound having an effect of preventing, ameliorating, or eliminating symptoms of cancer, an immune disease, or an infectious disease.

A better understanding of the present invention may be obtained through the following examples. These examples are merely set forth to illustrate the present invention, and are not to be construed as limiting the scope of the present invention, as will be apparent to those of ordinary skill in the art.

### Example 1: Preparation and culture of PBMC-derived CD3(-)CD56(+) NK cells

### Example 1-1: Preparation of NK cells

In order to obtain CD3(-) cells, the number of PBMCs (peripheral blood mononuclear cells, Zen-Bio. Inc., NC 27709, USA, Cat#: SER-PBMC-200-F) was measured using an ADAM-MC2 automatic cell counter (NanoEnTek, purchased by Cosmogenetech). The counted PBMCs were transferred to a new tube and then centrifuged at 300x g and 4°C for 10 minutes.

A CliniMACS buffer (pH 7.2) was prepared by supplementing a CliniMACS PBS/EDTA buffer (Miltenyi Biotec, Bergisch Gladbach, Germany, Cat#: 200-070-029) with 0.5% (v/v) of HSA (human serum albumin) and 1 mM of EDTA. 1×10⁷ PBMCs resulting from centrifugation were treated with 80 µL of the CliniMACS buffer and 20 µL of CD3 magnetic beads (Miltenyi biotech, 130-050-101) and the cell pellets were suspended, followed by reaction at 4°C for 15 minutes. For washing, 10 mL of a MACS buffer was added thereto, followed by centrifugation at 340x g and 4°C for 10 minutes, after which the supernatant was discarded, and the cell pellets were resuspended in 0.5 mL of the CliniMACS buffer. 2 mL of the MACS buffer was allowed to flow through an LD column (Miltenyi Biotec, Bergisch Gladbach, Germany, Cat#: 130-042-901), after which the resuspended cells were allowed to flow therethrough, so CD3(-) cells passing through the LD column were collected. Here, 2 mL of the CliniMACS buffer was allowed to flow therethrough three times so that the cells remaining in the LD column could be sufficiently separated, thereby obtaining CD3(-) cells, which were then placed in a new tube, followed by centrifugation at 340x g and 4°C for 10 minutes. Thereafter, the supernatant was removed, and 1×10⁷ cells were added with 80 µL of the MACS buffer and 20 µL of CD56 magnetic beads (Miltenyi biotech, Cat#: 130-050-401), followed by reaction at 4°C for 15 minutes. For washing, 10 mL of the CliniMACS buffer was added thereto, followed by centrifugation at 340x g and 4°C for 10 minutes, after which the supernatant was discarded, and the cell pellets were resuspended in 0.5 mL of the CliniMACS buffer. 3 mL of the CliniMACS buffer was allowed to flow through an LS column (Miltenyi Biotec, Bergisch Gladbach, Germany, Cat#: 130-042-901), after which the cell suspension was allowed to flow therethrough. Here, 2 mL of the CliniMACS buffer was allowed to flow therethrough three times so that the cells remaining in the LS column could be sufficiently separated. Thereafter, the LS column was separated on a magnetic stand, 5 mL of the CliniMACS buffer was added thereto, and pressure was applied with a piston to obtain CD3(-)CD56(+) NK cells.

The CD3 (-) CD56 (+) NK cells thus obtained were placed in a new tube and centrifuged at 340x g and 4°C for 10 minutes, after which the supernatant was removed, the cell pellets were suspended in a culture medium, and the cells were counted using a cell counter.

### Examples 1-2: Culture of NK cells

In order to prepare NK activation beads in the NK Cell Activation/Expansion Kit (Cat#: 130-112-968) (Miltenyi Biotec, Bergisch Gladbach, Germany), 100 µL of CD335 (NKp46)-biotin and 100 µL of CD2-biotin were placed in a 1.5 mL microtube, mixed, added with 500 µL of anti-biotin MACSiBead particles, mixed, added with 300 µL of a MACS buffer, and mixed at 2-8°C for 2 hours using a microtube rotator. Taking into consideration the number of CD3(-)CD56(+) cells, 5 µL of NK activation beads per 1×10⁶ cells were transferred into a new tube, and then 1 mL of an NK MACS medium (Cat#: 130-094-483) (Miltenyi Biotec, Bergisch Gladbach, Germany) was added thereto, followed by centrifugation at 300x g for 5 minutes. The supernatant was removed, after which NK activation beads were suspended by adding the NK MACs medium (Miltenyi Biotec, Bergisch Gladbach, Germany, Cat#: 130-094-483) based on 5 µL per 10⁶ NK cells. The NK cells were cultured for 21 days in the presence of 50 nM/mL GI-101 (GICELL Inc.) in a medium (Miltenyi Biotec, Bergisch Gladbach, Germany, Cat#: 130-094-483) supplemented with 5% human AB serum (MILAN Analytica AG, Rheinfelden, Switzerland Cat#: 000084).

### Example 2: Preparation and cryopreservation of composition for NK cell cryopreservation

### Example 2-1: Preparation of composition for NK cell cryopreservation

In order to prepare a composition for cryopreservation, the basic composition shown in Table 1 below was prepared by sequentially mixing 50% (v/v) of Plasma Solution A (HK inno.N), 5% (v/v) of DMSO (Avantor, CAT# 9033-04-01), and 20% (v/v) of albumin (Green Cross). Thereafter, as additional cryoprotectants, glucose (JW Pharmaceutical), PEG400 (Polyethylene Glycol 400 (USP-NF, BP, Ph. Eur. pure, pharma grade, PanReac AppliChem, CAT# 142436)), and trehalose (Spectrum, CAT# T9601) were added to reach the final concentrations shown in Table 1 below. Here, the cryoprotectant that was added was diluted with Plasma Solution A to a volume corresponding to 25% of the total composition and used. As a control group, a commercially available composition for cryopreservation, namely a CS10 medium (Table 2), was used.

**[Table 1]**

| | **Detailed composition** | | | | **Final concentration** | |
|---|---|---|---|---|---|---|
| | | | | | **%w/v** | **%v/v** |
| **Basic composition** | **Plasma Solution A (294 mOsm/L)** | **Sodium (Na)** | 140 | mmol/L | 0.1609286 | 50 |
| | | **Chloride (C1)** | 98 | mmol/L | 0.1737197 | |
| | | **Potassium (K)** | 5 | mmol/L | 0.009774575 | |
| | | **Magnesium (Mg)** | 3 | mmol/L | 0.0018 | |
| | | **Acetate** | 27 | mmol/L | 0.0810702 | |
| | | **Gluconate** | 23 | mmol/L | 0.225584 | |
| | **Human Serum Albumin** | | 0.2 | g/mL | 4 | 20 |
| | **DMSO** | | - | - | | 5 |
| **Cryoprotectant** | **Candidate 1-1** | **Glucose** | 0.05 | g/mL | 1.25 | 25 |
| | **Candidate 1-2** | **Glucose** | 0.1 | g/mL | 2.5 | 25 |
| | **Candidate 1-3** | **Glucose** | 0.2 | g/mL | 5 | 25 |
| | **Candidate 2-1** | **PEG 400** | 0.025 | g/mL | 0.625 | 25 |
| | **Candidate 2-2** | **PEG 400** | 0.1 | g/mL | 2.5 | 25 |
| | **Candidate 3-1** | **Trehalose** | 0.0684 | g/mL | 1.71 | 25 |
| | **Candidate 3-2** | **Trehalose** | 0.342 | g/mL | 8.55 | 25 |

**[Table 2]**

| | **Detailed composition** | | |
|---|---|---|---|
| **Comparative Example (CS10)** | **Sodium (Na)** | 100 | mmol/L |
| | **Chloride (C1)** | 17.1 | mmol/L |
| | **Potassium (K)** | 42.5 | mmol/L |
| | **Magnesium (Mg)** | 5 | mmol/L |
| | **Calcium (Ca²⁺)** | 0.05 | mmol/L |
| | **H2PO4-** | 10 | mmol/L |
| | **HCO3-** | 5 | mmol/L |
| | **HEPES** | 25 | mmol/L |
| | **Lactobionate** | 100 | mmol/L |
| | **Sucrose** | 20 | mmol/L |
| | **Glucose** | 5 | mmol/L |
| | **Adenosine** | 2 | mmol/L |
| | **Glutathione** | 3 | mmol/L |
| | **Dextran-40** | 6 | % |
| | **DMSO** | 10 | % |
| | **Tonicity** | 360 | mOsm/L |

### Example 2-2: Cryopreservation of NK cells

The NK cells prepared in Example 1 were suspended at a concentration of 1×10⁶ cells/mL in the composition for NK cell cryopreservation to obtain an NK cell cryopreservation formulation, which was then dispensed in an amount of 1 mL into respective vials, stored in a cryogenic freezer for one day, and transferred to LN₂ for cryopreservation.

### Example 3: Measurement of yield and viability of cryopreservation formulation candidate after thawing

The number of viable cells and the viability of NK cells cryopreserved for 4 weeks were measured using an ADAM cell counter Accustain kit (DigitalBio) according to the manufacturer's protocol.

Specifically, the frozen NK cells were thawed and then diluted with PBS to a concentration of 1×10⁶ cell/mL, and 14 µL thereof was dispensed into each of two wells of a round-bottomed 96-well plate. The cultured cells were counted, and 14 µL of a T solution for measuring the total number of cells was added to one of the two wells and mixed with the diluted cells, and 14 µL of the resulting mixture was taken and placed in the T panel of a counter chip. Meanwhile, 14 µL of an N solution for measuring the number of dead cells was added to the remaining well and mixed with the diluted cells, and 14 µL of the resulting mixture was taken and placed in the N panel of the counter chip. The chip was inserted into an ADAM counter and the number of viable cells was measured, and the results thereof are shown in FIGS. 1A and 1B.

As results confirming the number of viable cells and the viability of NK cells frozen for 4 weeks according to Example 1, as shown in FIGS. 1A and 1B, based on the NK cells cryopreserved in the cryopreservation formulation using the control group (CS10), it was confirmed that the cryopreservation formulations using the candidates of Table 1 showed generally the same level of cell yield and viability. Among them, the yields of candidates 1-2 and 1-3, candidates 2-1 and 2-2, and candidate 3-2 appeared to be high, and candidate 2-1 including PEG as a stabilizer exhibited the highest yield.

### Example 4: Identification of surface markers of cryopreserved NK cells

The NK cells cryopreserved using each of the eight cryopreservation formulations were suspended in 10 mL of an NK MACS medium (Miltenyi), followed by centrifugation at 1,300 rpm for 5 minutes. The supernatant was removed, and the cell pellets were suspended in 10 mL of the NK MACS medium.

An FACS buffer was prepared by supplementing PBS with 3% (v/v) of FBS, 10 mM of EDTA, 20 mM of HEPES, 10 ug/mL of PolymyxinB, 100 U/mL of penicillin, 100 pg/mL of streptomycin, and 1 mM of sodium pyruvate. Thereafter, the cells were diluted with the FACS buffer to a concentration of 2×10⁶ cells/mL, verified using a cell counter. 100 µL of the diluted cell solution was added to a 96-well plate and then centrifuged at 1,300 rpm for 5 minutes, after which the supernatant was removed, and the cell pellets were suspended in 200 µL of the FACS buffer. After centrifugation at 1,300 rpm for 5 minutes, 50 µL of an FC blocking solution prepared at a 1X concentration from a 200X Fc block (BioLegend, San Diego, CA, USA) solution using the FACS buffer was added to each well and allowed to stand on ice for 10 minutes.

An FITC-labeled anti-human CD3 antibody (FITC anti-human CD3 Antibody (Clone UCHT1)), a PE-Cy7-labeled anti-human CD56 antibody (PE/Cyanine7 anti-human CD56 (NCAM) Antibody (Clone 5.1H11)), a BV421-labeled anti-human CD14 antibody (Brilliant Violet 421^{™} anti-human CD14 Antibody (Clone 63D3)), a BV480-labeled anti-human CD19 antibody (BV480 Mouse Anti-Human CD19 (Clone SJ25C1)), a PE-labeled anti-human CD16 antibody (PE anti-human CD16 Antibody (Clone 3G8)), a BV711-labeled anti-human NKp46 antibody (Brilliant Violet 711^{™} anti-human CD335 (NKp46) Antibody (Clone 9E2)), a BV650-labeled anti-human NKG2D antibody (BD Horizon^{™} BV650 Mouse Anti-Human CD314 (NKG2D) (Clone 1D11)), a BV605-labeled anti-human NKG2C antibody (BV605 Mouse Anti-Human CD159c (NKG2C) (Clone 134591)), a BV785-labeled anti-human CXCR3 antibody (Brilliant Violet 785^{™} anti-human CD183 (CXCR3) Antibody (Clone EH12.2H7)), a PE-labeled anti-human CCR5 antibody (PE anti-human CD195 (CCR5)) Antibody (Clone J418F1)), and a BV650-labeled anti-human DNAM-1 antibody (BD OptiBuild^{™} BV650 Mouse Anti-Human CD226 (Clone DX11)) were added to each well along with 50 µL of the FACS buffer, allowed to react at 4°C for 20 minutes, added with a further 100 µL of the FACS buffer, and then centrifuged at 1,300 rpm for 5 minutes. Here, markers with the same fluorescence were stained after separation of the panel. The materials such as media and buffers that were used are listed in Table 3 below, and information on the markers and antibodies is listed in Table 4 below.

**[Table 3]**

| **Material** | **Cat#** | **Producer** |
|---|---|---|
| NK MACS medium | 130-114-429 | Miltenyi |
| PBS | LB001-02 | Welgene |
| 500mM EDTA | ML005-01 | Welgene |
| 1M HEPES | BB001-01 | Welgene |
| 25 mg/ml PolymyxinB | 92283-10ML | Sigma |
| Penicillin & Streptomycin (100x) | LS202-02 | Welgene |
| 100 mM sodium pyruvate | LS013-01 | Welgene |
| Human TruStain FcX^{™} (Fc Receptor Blocking Solution) | 422302 | BioLegend |

**[Table 4]**

| | **Target** | **Color** | **Clone** | **Producer** | **Cat#** |
|---|---|---|---|---|---|
| **Purity marker** | CD3 | FITC | UCHT1 | BioLegend | 300406 |
| | CD56 | PE/Cyanine7 | 5.1H11 | BioLegend | 362510 |
| | CD14 | BV421 | 63D3 | BioLegend | 367144 |
| | CD19 | BV480 | SJ25C1 | BD | 566103 |
| **Activation marker** | CD16 | PE | 3G8 | BioLegend | 302008 |
| | NKp46 | BV711 | 9E2 | BioLegend | 331936 |
| | NKG2D | BV650 | 1D11 | BD | 563408 |
| | NKG2C | BV605 | 134591 | BD | 748166 |
| | CXCR3 | BV785 | G025H7 | BioLegend | 353738 |
| | CCR5 | PE | J418F1 | BioLegend | 359106 |
| | DNAM-1 | BV650 | DX11 | BD | 742496 |

The supernatant was removed, and the cell pellets were suspended in 200 µL of the FACS buffer, centrifuged at 1,300 rpm for 5 minutes, and then resuspended in 200 µL of the FACS buffer. The phenotype of the cells was confirmed using a flow cytometer (Cytek^{®} Aurora, Cytek, Fremont, CA, USA).

Consequently, as shown in FIG. 2, expression of surface markers indicating the activity or inhibition of NK cells cryopreserved in the control group (CS10) and the candidate 1-1 to 3-2 formulations shown in Table 1 was found to be at the same level.

### Example 5: Identification of cytotoxicity markers of cryopreserved NK cells

The NK cells cryopreserved using each of the eight cryopreservation formulations were suspended in 10 mL of an NK MACS medium (Miltenyi), followed by centrifugation at 1,300 rpm for 5 minutes. The supernatant was removed, and the cell pellets were suspended in 10 mL of the NK MACS medium.

An FACS buffer was prepared by supplementing PBS with 3% (v/v) of FBS, 10 mM of EDTA, 20 mM of HEPES, 10 µg/mL of PolymyxinB, 100 U/mL of penicillin, 100 pg/mL of streptomycin, and 1 mM of sodium pyruvate. Thereafter, the cells were diluted with the NK MACS medium to a concentration of 2×10⁶ cells/mL, verified using a cell counter. 100 µL of the diluted cell solution was added to a 96-well plate, and a 500X stimulation cocktail (eBioscience^{™} Cell Stimulation Cocktail (plus protein transport inhibitors) (500X)) was diluted to a 2X concentration with the NK MACS medium. 100 µL of the prepared stimulation solution was added to each well, followed by culture in an incubator at 37°C and 5% CO₂ for 4 hours.

After culture for 4 hours, centrifugation was performed at 1,300 rpm for 5 minutes, after which the supernatant was removed, and the cell pellets were suspended in 200 µL of the FACS buffer. After centrifugation at 1,300 rpm for 5 minutes, 50 µL of an Fc blocking solution prepared at a 1X concentration from a 200X Fc block (BioLegend, San Diego, CA, USA) solution using the FACS buffer was added to each well and allowed to stand on ice for 10 minutes.

A PerCP-labeled anti-human CD3 antibody (PerCP anti-human CD3 Antibody (Clone HIT3a)), an APC-Cy7-labeled anti-human CD56 antibody (APC/Cyanine7 anti-human CD56 (NCAM) Antibody (Clone 5.1H11)), a BV421-labeld anti-human Tim-3 antibody (Brilliant Violet 421^{™} anti-human CD366 (Tim-3) Antibody (Clone F38-2E2)), a BV785-labeled anti-human CD16 antibody (Brilliant Violet 785^{™} anti-human CD16 Antibody (Clone 3G8)), and a PE-labeled anti-human PD-1 antibody (PE anti-human CD279 (PD-1) Antibody (Clone A17188B)) were added to each well along with 50 µL of the FACS buffer, allowed to react at 4°C for 20 minutes, added with a further 100 µL of the FACS buffer, and then centrifuged at 1,300 rpm for 5 minutes.

The supernatant was removed, and the cell pellets were suspended in 200 µL of the FACS buffer and then centrifuged at 1,300 rpm for 5 minutes. The supernatant was removed, and the cell pellets were suspended in 100 µL of a BD Cytofix/Cytoperm^{™} Fixation and Permeabilization Solution and then allowed to stand on ice for 30 minutes.

A BD Perm/Wash^{™} buffer was diluted to a 1X concentration with DW and allowed to react for 30 minutes, after which 100 µL of the prepared wash buffer was added to each well, followed by centrifugation at 1,300 rpm for 5 minutes. The supernatant was removed, and the cell pellets were added with 200 µL of the wash buffer and then centrifuged at 1,300 rpm for 5 minutes.

An FITC-labeled anti-human IFN-γ antibody (FITC anti-human IFN-γ Antibody (Clone 4S.B3)), a PE-Cy7-labeled anti-human Granzyme B antibody (PE/Cyanine7 anti-human/mouse Granzyme B Recombinant Antibody (Clone QA16A02)), and an APC-labeled anti-human Perforin antibody (APC anti-human Perforin Antibody (Clone B-D48)) were added to each well along with 100 µL of the wash buffer, allowed to react at 4°C for 20 minutes, added with a further 100 µL of the wash buffer, and then centrifuged at 1,300 rpm for 5 minutes.

The supernatant was removed, and the cell pellets were suspended in 200 µL of the wash buffer, centrifuged at 1,300 rpm for 5 minutes, and then resuspended in 200 µL of the FACS buffer. Expression of toxic markers of the cells was confirmed using a flow cytometer (Cytek^{®} Aurora, Cytek, Fremont, CA, USA).

The materials such as media and buffers that were used are listed in Table 5 below, and information on the markers and antibodies that were used is listed in Table 6 below.

**[Table 5]**

| **Material** | **Cat#** | **Producer** |
|---|---|---|
| ***NK* MACS medium** | 130-114-429 | Miltenyi |
| **PBS** | LB001-02 | Welgene |
| **500mM EDTA** | ML005-01 | Welgene |
| **1M HEPES** | BB001-01 | Welgene |
| **25 mg/ml PolymyxinB** | 92283-10ML | Sigma |
| **Penicillin&Streptomycin (100x)** | LS202-02 | Welgene |
| **100 mM sodium pyruvate** | LS013-01 | Welgene |
| **Human TruStain FcX^{™} (Fc Receptor Blocking Solution)** | 422302 | BioLegend |
| **eBioscience^{™} Cell Stimulation Cocktail (plus protein transport inhibitors) (500X)** | 00-4975-03 | eBioscience |
| **Fixation and Permeabilization Solution** | 554722 | BD |
| **BD Perm/Wash^{™} buffer** | 554723 | BD |

**[Table 6]**

| | **Target** | **Color** | **Clone** | **Producer** | **Cat#** |
|---|---|---|---|---|---|
| **Purity marker** | CD3 | PerCP | HIT3a | BioLegend | 300326 |
| | CD56 | APC/Cyanine7 | 5.1H11 | BioLegend | 362512 |
| | CD16 | BV785 | 3G8 | BioLegend | 302046 |
| **Inhibition marker** | Tim-3 | BV421 | F38-2E2 | BioLegend | 345008 |
| | PD-1 | PE | A17188B | BioLegend | 621608 |
| **Cytotoxicity marker** | IFN-γ | FITC | 4S.B3 | BioLegend | 502506 |
| | Granzyme B | PE/Cyanine7 | QA16A02 | BioLegend | 372214 |
| | Perforin | APC | B-D48 | BioLegend | 353312 |

Consequently, as shown in FIG. 3, expression of IFN-γ, perforin, and Granzyme B, which are known degranulation materials, was found to be at the same level in the NK cells cryopreserved in Comparative Example (CS10) and the candidate 1-1 to 3-2 formulations shown in Table 2.

### Example 6: Confirmation of ability of cryopreserved NK cells to kill cancer cells

Information on the materials that were used in Example 5 is shown in Table 7 below.

**[Table 7]**

| **Cat No.** | **Manufacturer** | **Product** |
|---|---|---|
| **LM011-01** | Welgene | RPMI 1640 Medium |
| **C3099** | Invitrogen | Calcein, AM |
| **3799** | Corning | 96 well Cell Culture Plate (round bottom) |
| **655209** | Greiner Bio-One | Flat bottom, 96-well Plate, Black, PP |
| **T9284-100ML** | Sigma Aldrich | Triton X-100 |
| **23050** | SPL | Reservoir, 50ml |

### Example 6-1: Preparation of target cells

The target cells (K562, BT474, MDA-MB-231) were obtained, centrifuged at 1,300 rpm for 5 minutes, and then suspended at a concentration of 1×10⁶ cells/mL in an RPMI 1640 medium (Welgene, LM011-01). The 1×10⁶ target cells thus prepared were stained with 30 µL of Calcein-AM. After culture in an incubator (37°C, 5% CO₂) for 1 hour in the dark, the cells were washed twice with 10 mL of the RPMI 1640 medium. The supernatant was discarded, and the cell pellets were suspended in the RPMI 1640 medium to obtain a target cell suspension at a concentration of 1×10⁵ cells/mL.

### Example 6-2: Preparation of effector cells

5×10⁶ NK cells cryopreserved for 4 weeks in each cryopreservation formulation were obtained, centrifuged at 1,300 rpm for 10 minutes, and then suspended at a concentration of 1×10⁶ cells/mL in an RPMI 1640 medium (Welgene, LM011-01). The cells were dispensed into a 96-well plate at the E:T ratios (10:1, 3:1, 1:1, and 0.3:1) shown in Table 8 below. 100 µL of the RPMI 1640 medium was added to a well without NK cells to obtain a "Spontaneous Value", and a "Maximum Value" was determined by addition of 100 µL of 2% Triton X-100/DPBS. In addition, "MM" was determined by addition of 200 µL of the RPMI 1640 medium.

**[Table 8]**

| **E:T ratio** | **NK cells** | **RPMI Media** | **NK cell concentration** |
|---|---|---|---|
| **E10** | Resuspended NK cells | 5 mL | 1×10⁶ cells/mL |
| **E3** | 1.5 mL of E10 | 3.5 mL | 0.3×10⁶ cells/mL |
| **E1** | 500 µL of E10 | 4.5 mL | 0.1×10⁶ cells/mL |
| **E0.3** | 500 µL of E3 | 4.5 mL | 0.03×10⁶ cells/mL |

### Example 6-3: Co-culture

100 µL of the stained target cells of Example 6-1 was dispensed into each well of the 96-well plate prepared in Example 6-2, followed by co-culture at 37°C and 5% CO₂ for 4 hours in the dark. After co-culture, centrifugation was performed at 2000 rpm for 3 minutes, after which 100 µL of the supernatant was obtained and dispensed into a new flat-bottomed 96-well plate, and fluorescence was measured at wavelengths of 485 nm and 535 nm (FIGS. 4A-4D to 6A-6D) .

Consequently, as shown in FIGS. 4A-4D to 6A-6D, the ability of NK cells to kill cancer cells was evaluated to be significantly improved when using candidate 1-1 with glucose, candidate 2-1 with PEG, and candidate 3-1 with trehalose as cryopreservation formulations, compared to the control group (CS10). The ability thereof varied slightly depending on the types of target cells, but showed a similar tendency overall, and it was confirmed that the cancer-cell-killing ability of NK cells cryopreserved in the candidate cryopreservation formulation of the present invention was vastly superior.

When the results of the above examples are summarized, candidates 1-1 and 1-2, candidates 2-1 and 2-2, and candidate 3-1 of the present invention exhibited cell surface marker expression (FIG. 2) and NK cell degranulation ability (FIG. 3) at the same level as the formulation using the conventional cryopreservation medium (CS10), even in low-concentration DMSO not including HEPES (hydroxyethyl piperazine ethane sulfonic acid) or HES (hydroxyethyl starch), and also manifested a high cell yield (FIG. 1A) and vastly superior ability of NK cells to kill cancer cells in practice (FIGS. 4A-4D to 6A-6D). Among them, candidate 2-1 showed the highest yield when thawed, and stably exhibited high ability to kill cancer cells.

### Example 7: Preparation and cryopreservation of composition for NK cell cryopreservation depending on molecular weight of PEG

### Example 7-1: Preparation of composition for NK cell cryopreservation

In order to prepare a composition for cryopreservation, the basic composition shown in Table 1 was prepared by sequentially mixing 50% (v/v) of Plasma Solution A (HK inno.N), 5% (v/v) of DMSO (Avantor, CAT# 9033-04-01), and 20% (v/v) of albumin (Green Cross). Then, as additional cryoprotectants, PEG400 (Polyethylene Glycol 400 (USP-NF, BP, Ph. Eur. pure, pharma grade, PanReac AppliChem, CAT# 142436), PEG4000 (Sigma, CAT#81242), and PEG20000 (Sigma, CAT) #81275) were added to the final concentrations shown in Table 1. Here, the cryoprotectant that was added was diluted with Plasma Solution A to a volume corresponding to 25% of the total composition and used. As a control group, a composition including only basic components without adding PEG, like Comparative Example 2, was used.

**[Table 9]**

| | **Detailed composition** | | | | **Final concentration** | |
|---|---|---|---|---|---|---|
| | | | | | **%w/v** | **%v/v** |
| **Basic composition** | **Plasma Solution A (294 mOsm/L)** | **Sodium (Na)** | 140 | mmol/L | 0.1609286 | 50 |
| | | **Chloride (Cl)** | 98 | mmol/L | 0.1737197 | |
| | | **Potassium (K)** | 5 | mmol/L | 0.009774575 | |
| | | **Magnesium (Mg)** | 3 | mmol/L | 0.0018 | |
| | | **Acetate** | 27 | mmol/L | 0.0810702 | |
| | | **Gluconate** | 23 | mmol/L | 0.225584 | |
| | **Human Serum Albumin** | | 0.2 | g/mL | 4 | 20 |
| | **DMSO** | | - | - | | 5 |
| **Cryoprotectant** | **Comparative Example 2 without PEG** | - | - | - | - | - |
| | **Candidate 2-3** | **PEG 400** | 0.09 | g/mL | 2.25 | 25 |
| | **Candidate 2-4** | **PEG 4000** | 0.09 | g/mL | 2.25 | 25 |
| | **Candidate 2-5** | **PEG 4000** | 0.009 | g/mL | 0.225 | 25 |
| | **Candidate 2-6** | **PEG 20000** | 0.09 | g/mL | 2.25 | 25 |
| | **Candidate 2-7** | **PEG 20000** | 0.0018 | g/mL | 0.045 | 25 |

### Example 7-2: Cryopreservation of NK cells

The NK cells prepared in Example 1 were suspended at a concentration of 1×10⁶ cells/mL in the composition for NK cell cryopreservation to obtain an NK cell cryopreservation formulation, which was then dispensed in an amount of 1 mL into respective vials, stored in a cryogenic freezer for one day, and transferred to LN₂ for cryopreservation.

### Example 8: Confirmation of yield and viability of composition for cryopreservation depending on molecular weight of PEG after thawing

The number of viable cells and the viability of NK cells frozen for 2 weeks were measured using an ADAM cell counter Accustain kit (DigitalBio) according to the manufacturer's protocol.

Specifically, the frozen NK cells were thawed and then diluted to a concentration of 1×10⁶ cell/mL with PBS, and 13 µL thereof was dispensed into each of two wells of a round-bottomed 96-well plate. The cultured cells were counted, after which 13 µL of a T solution for measuring the total number of cells was added to one of the two wells and mixed with the diluted cells, and 13 µL of the resulting mixture was taken and placed in the T panel of a counter chip. Meanwhile, 13 µL of an N solution for measuring the number of dead cells was added to the remaining well and mixed with the diluted cells, and 13 µL of the resulting mixture was taken and placed in the N panel of the counter chip.

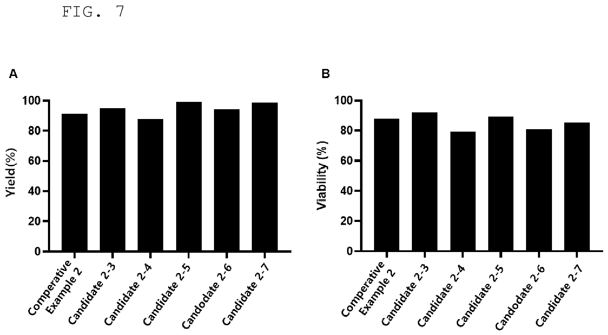

The chip was inserted into an ADAM counter and the number of viable cells was measured, and the results thereof are shown in FIGS. 7A and 7B. As is apparent from the results of FIGS. 7A and 7B, the yield and viability of candidates 2-3 to 2-7 and Comparative Example 2 after thawing were found to be at the same level.

### Example 9: Identification of surface markers of NK cells in composition for cryopreservation depending on molecular weight of PEG

The NK cells frozen using each of the six cryopreservation formulations shown in Table 9 were suspended in 10 mL of an NK MACS medium (Miltenyi), followed by centrifugation at 1,300 rpm for 5 minutes. The supernatant was removed, and the cell pellets were suspended in 10 mL of the NK MACS medium.

An FACS buffer was prepared by supplementing PBS with 3% (v/v) of FBS, 10 mM of EDTA, 20 mM of HEPES, 10 µg/mL of PolymyxinB, 100 U/mL of penicillin, 100 pg/mL of streptomycin, and 1 mM of sodium pyruvate. Thereafter, the cells were diluted with the FACS buffer to a concentration of 2×10⁶ cells/mL, verified using a cell counter. 100 µL of the diluted cell solution was added to a 96-well plate and centrifuged at 1,300 rpm for 5 minutes, after which the supernatant was removed, and the cell pellets were suspended in 200 µL of the FACS buffer. After centrifugation at 1,300 rpm for 5 minutes, 50 µL of an Fc blocking solution prepared at a 1X concentration from a 200X Fc block (BioLegend, San Diego, CA, USA) solution using the FACS buffer was added to each well and allowed to stand on ice for 10 minutes.

A PerCP-labeled anti-human CD3 antibody (PerCP anti-human CD3 Antibody (Clone HIT3a)), a PE-Cy7 labeled anti-human CD56 antibody (PE-Cy^{™}7 Mouse Anti-Human CD56 (NCAM-1) (Clone B159)), an Alexa Fluor^{®} 700-labeled anti-human CD14 antibody (Alexa Fluor^{®} 700 Mouse Anti-Human CD14 (Clone M5E2)), a BV786-labeled anti-human CD19 antibody (BV786 Mouse Anti-Human CD19 (Clone SJ25C1)), an APC-H7-labeled anti-human CD16 antibody (APC-H7 Mouse Anti-Human CD16 (Clone 3G8)), a BV711-labeled anti-human NKp46 antibody (Brilliant Violet 711^{™} anti-human CD335 (NKp46 Antibody (Clone 9E2)), a PE-CF594-labeled anti-human NKG2D antibody (PE-CF594 Mouse Anti-Human CD314 (NKG2D) (Clone 1D11)), a BV421-labeled anti-human NKG2C antibody (BV421 Mouse Anti-Human CD159c (NKG2C) (Clone 134591)), a BV605-labeled anti-human CXCR3 antibody (BV605 Mouse Anti-Human CD183 (Clone 1C6/CXCR3)), an APC-labeled anti-human CCR5 antibody (APC Mouse Anti-Human CD195 (Clone 3A9)), and an Alexa Fluor^{®} 647-labeled anti-human DNAM-1 antibody (Alexa Fluor^{®} 647 Mouse Anti-Human CD226 (Clone DX11)) were added to each well along with 50 µL of the FACS buffer, allowed to react at 4°C for 20 minutes, added with a further 100 µL of the FACS buffer, and then centrifuged at 1,300 rpm for 5 minutes. Here, markers with the same fluorescence were stained after separation of the panel. The materials such as media and buffers that were used are listed in Table 10 below, and information on the markers and antibodies is listed in Table 11 below.

**[Table 10]**

| **Material** | **Cat#** | **Producer** |
|---|---|---|
| NK MACS medium | 130-114-429 | Miltenyi |
| PBS | LB001-02 | Welgene |
| 500mM EDTA | ML005-01 | Welgene |
| 1M HEPES | BB001-01 | Welgene |
| 25 mg/ml PolymyxinB | 92283-10ML | Sigma |
| Penicillin&Streptomycin (100x) | LS202-02 | Welgene |
| 100 mM sodium pyruvate | LS013-01 | Welgene |
| Human TruStain FcX^{™} (Fc Receptor Blocking Solution) | 422302 | BioLegend |

**[Table 11]**

| | **Target** | **Color** | **Clone** | **Producer** | **Cat#** |
|---|---|---|---|---|---|
| **Purity marker** | CD3 | PerCP | HIT3a | Biolegend | 300326 |
| | CD56 | PE/Cyanine7 | B159 | BD | 557747 |
| | CD14 | Alexa Fluor^{®} 700 | M5E2 | BD | 557923 |
| | CD19 | BV7 8 6 | SJ25C1 | BD | 563325 |
| **Activation marker** | CD16 | APC-H7 | 3G8 | BD | 560195 |
| | DNAM-1 | Alexa Fluor^{®} 647 | DX11 | BD | 564797 |
| | CCR5 | APC | 3A9 | BD | 550856 |
| | CXCR3 | BV605 | 1C6/CXCR3 | BD | 564032 |
| | NKG2D | PE-CF594 | 1D11 | BD | 562498 |
| | NKp46 | BV711 | 9E2 | Biolegend | 331936 |
| | NKG2C | BV421 | 134591 | BD | 748169 |

The supernatant was removed, and the cell pellets were suspended in 200 µL of the FACS buffer, centrifuged at 1,300 rpm for 5 minutes, and then resuspended in 200 µL of the FACS buffer. The phenotype of the cells was confirmed using a flow cytometer (Cytek^{®} Aurora, Cytek, Fremont, CA, USA).

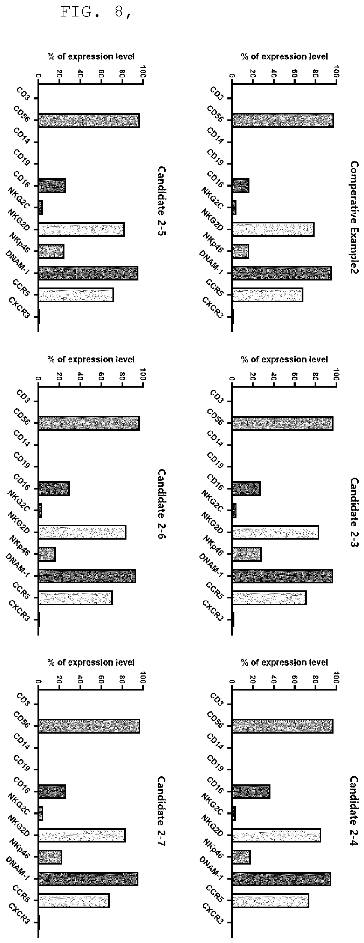

Consequently, as shown in FIG. 8, expression of the cell surface markers of candidates 2-3 to 2-7 and Comparative Example 2 was found to be at the same level.

### Example 10: Identification of cytotoxicity markers of NK cells in composition for cryopreservation depending on molecular weight of PEG

The NK cells frozen using each of the six cryoprotectants shown in Table 9 were suspended in 10 mL of an NK MACS medium (Miltenyi), followed by centrifugation at 1,300 rpm for 5 minutes. The supernatant was removed, and the cell pellets were suspended in 10 mL of the NK MACS medium.

An FACS buffer was prepared by supplementing PBS with 3% (v/v) of FBS, 10 mM of EDTA, 20 mM of HEPES, 10 µg/mL of PolymyxinB, 100 U/mL of penicillin, 100 pg/mL of streptomycin, and 1 mM of sodium pyruvate. Thereafter, the cells were diluted with the NK MACS medium to a concentration of 2×10⁶ cells/mL, verified using a cell counter. 100 µL of the diluted cell solution was added to a 96-well plate, and a 500X stimulation cocktail (eBioscience^{™} Cell Stimulation Cocktail (plus protein transport inhibitors) (500X)) was diluted to a 2X concentration with the NK MACS medium. 100 µL of the prepared stimulation solution was added to each well, followed by culture in an incubator at 37°C and 5% CO₂ for 4 hours.

After culture for 4 hours, centrifugation was performed at 1,300 rpm for 5 minutes, after which the supernatant was removed, and the cell pellets were suspended in 200 µL of the FACS buffer. After centrifugation at 1,300 rpm for 5 minutes, 50 µL of an Fc blocking solution prepared at a 1X concentration from a 200X Fc block (BioLegend, San Diego, CA, USA) solution using the FACS buffer was added to each well and allowed to stand on ice for 10 minutes.

A PerCP-labeled anti-human CD3 antibody (PerCP anti-human CD3 Antibody (Clone HIT3a)), an APC-Cy7-labeled anti-human CD56 antibody (APC/Cyanine7 anti-human CD56 (NCAM) Antibody (Clone 5.1H11)), a BV421-labeled anti-human Tim-3 antibody (Brilliant Violet 421^{™} anti-human CD366 (Tim-3) Antibody (Clone F38-2E2)), a BV785-labeled anti-human CD16 antibody (Brilliant Violet 785^{™} anti-human CD16 Antibody (Clone 3G8)), and a PE-labeled anti-human PD-1 antibody (PE anti-human CD279 (PD-1) Antibody (Clone A17188B)) were added to each well along with 50 µL of the FACS buffer, allowed to react at 4°C for 20 minutes, added with a further 100 µL of the FACS buffer, and then centrifuged at 1,300 rpm for 5 minutes.

The supernatant was removed, and the cell pellets were suspended in 200 µL of the FACS buffer and then centrifuged at 1,300 rpm for 5 minutes. The supernatant was removed again, and the cell pellets were suspended in 100 µL of a BD Cytofix/Cytoperm^{™} Fixation and Permeabilization Solution and then allowed to stand on ice for 30 minutes.

A BD Perm/Wash^{™} buffer was diluted to a 1X concentration with DW and allowed to react for 30 minutes, after which 100 µL of the prepared wash buffer was added to each well, followed by centrifugation at 1,300 rpm for 5 minutes. The supernatant was removed, and the cell pellets were added with 200 µL of the wash buffer and then centrifuged for 5 minutes at 1,300 rpm.

An FITC-labeled anti-human IFN-γ antibody (FITC anti-human IFN-γ Antibody (Clone 4S.B3)), a PE-Cy7-labeled anti-human Granzyme B antibody (PE/Cyanine7 anti-human/mouse Granzyme) B Recombinant Antibody (Clone QA16A02)), and an APC-labeled anti-human Perforin antibody (APC anti-human Perforin Antibody (Clone B-D48)) were added to each well along with 100 µL of the wash buffer, allowed to react at 4°C for 20 minutes, added with a further 100 µL of the wash buffer, and then centrifuged at 1,300 rpm for 5 minutes.

The supernatant was removed, and the cell pellets were suspended in 200 µL of the wash buffer, centrifuged at 1,300 rpm for 5 minutes, and then resuspended in 200 µL of the FACS buffer. Expression of cytotoxicity markers in the cells was confirmed using a flow cytometer (Cytek^{®} Aurora, Cytek, Fremont, CA, USA).

The materials such as media and buffers that were used are listed in Table 12 below, and information on the markers and antibodies that were used is listed in Table 13 below.

**[Table 12]**

| **Material** | **Cat#** | **Producer** |
|---|---|---|
| **NK MACS medium** | 130-114-429 | Miltenyi |
| **PBS** | LB001-02 | Welgene |
| **500mM EDTA** | ML005-01 | Welgene |
| **1M HEPES** | BB001-01 | Welgene |
| **25 mg/ml PolymyxinB** | 92283-10ML | Sigma |
| **Penicillin&Streptomycin (100x)** | LS202-02 | Welgene |
| **100 mM sodium pyruvate** | LS013-01 | Welgene |
| **Human TruStain FcX^{™} (Fc Receptor Blocking Solution)** | 422302 | BioLegend |
| **eBioscience^{™} Cell Stimulation Cocktail (plus protein transport inhibitors) (500X)** | 00-4975-03 | eBioscience |
| **Fixation and Permeabilization Solution** | 554722 | BD |
| **BD Perm/Wash^{™} buffer** | 554723 | BD |

**[Table 13]**

| | **Target** | **Color** | **Clone** | **Producer** | **Cat#** |
|---|---|---|---|---|---|
| **Inhibition marker** | Tim-3 | BV421 | F38-2E2 | BioLegend | 345008 |
| | PD-1 | PE | A17188B | BioLegend | 621608 |
| **Cytotoxicity marker** | IFN-γ | FITC | 4S.B3 | BioLegend | 502506 |
| | Granzyme B | PE/Cyanine7 | QA16A02 | BioLegend | 372214 |
| | Perforin | APC | B-D48 | Biolegend | 353312 |

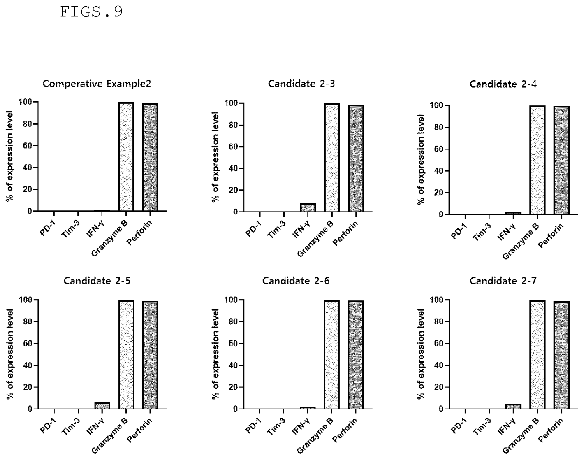

Consequently, as shown in FIG. 9, expression of the cytotoxicity markers of candidates 2-3 to 2-7 and Comparative Example 2 was found to be at the same level.

### Example 11: Confirmation of cancer-cell-killing ability of NK cells in composition for cryopreservation depending on molecular weight of PEG

Information on the materials that were used in Example 11 is shown in Table 14 below.

**[Table 14]**

| **Cat No.** | **Manufacturer** | **Product** |
|---|---|---|
| **LM011-01** | Welgene | RPMI 1640 Medium |
| **C3099** | Invitrogen | Calcein, AM |
| **3799** | Corning | 96 well Cell Culture Plate (round bottom) |
| **655209** | Greiner Bio-One | Flat bottom, 96-well Plate, Black, PP |
| **T9284-100ML** | Sigma Aldrich | Triton X-100 |
| **23050** | SPL | Reservoir, 50ml |

### Example 11-1: Preparation of target cells

The target cells (K562, DLD-1) were obtained, centrifuged at 1,300 rpm for 5 minutes, and then suspended at a concentration of 1×10⁶ cells/mL in an RPMI 1640 medium (Welgene, LM011-01). The 1×10⁶ target cells thus prepared were stained with 30 µL of Calcein-AM. After culture in an incubator (37°C, 5% CO₂) for 1 hour in the dark, the cells were washed twice with 10 mL of the RPMI 1640 medium. The supernatant was discarded, and the cell pellets were suspended in the RPMI 1640 medium to obtain a target cell suspension at a concentration of 1×10⁵ cells/mL.

### Example 11-2: Preparation of effector cells

The 5×10⁶ NK cells frozen for 4 weeks in each cryopreservation formulation were obtained, centrifuged at 1,300 rpm for 10 minutes, and then suspended at a concentration of 1×10⁶ cells/mL in an RPMI 1640 medium (Welgene, LM011-01). These cells were dispensed into a 96-well plate at the E:T ratios (10:1, 3:1, 1:1, and 0.3:1) shown in Table 15 below. 100 µL of the RPMI 1640 medium was added to a well without NK cells to obtain a "Spontaneous Value", and a "Maximum Value" was determined through addition of 100 µL of 2% Triton X-100/DPBS. "MT" was determined by repeatedly adding 100 µL of the RPMI 1640 medium and 100 µL of a 2% Triton X-100/DPBS three times to each well. Also, "MM" was determined by addition of 200 µL of the RPMI 1640 medium.

**[Table 15]**

| **E:T ratio** | **NK cells** | **RPMI Media** | **NK cell concentration** |
|---|---|---|---|
| **E10** | Resuspended NK cells | 5 mL | 1×10⁶ cells/mL |
| **E3** | 1.5 mL of E10 | 3.5 mL | 0.3×10⁶ cells/mL |
| **E1** | 500 µL of E10 | 4.5 mL | 0.1×10⁶ cells/mL |
| **E0.3** | 500 µL of E3 | 4.5 mL | 0.03×10⁶ cells/mL |

### Examples 11-3: Co-culture

100 µL of the stained target cells of Example 11-1 was dispensed into each well of the 96-well plate prepared in Example 11-2, followed by co-culture at 37°C and 5% CO₂ for 4 hours in the dark. After co-culture, centrifugation was performed at 2000 rpm for 3 minutes, after which 100 µL of the supernatant was obtained and dispensed into a new flat-bottomed 96-well plate, and fluorescence was measured at wavelengths of 485 nm and 535 nm.

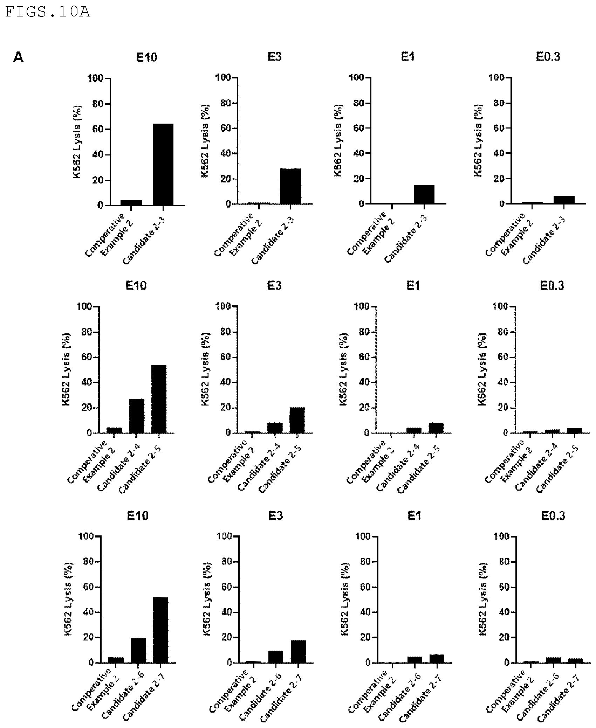

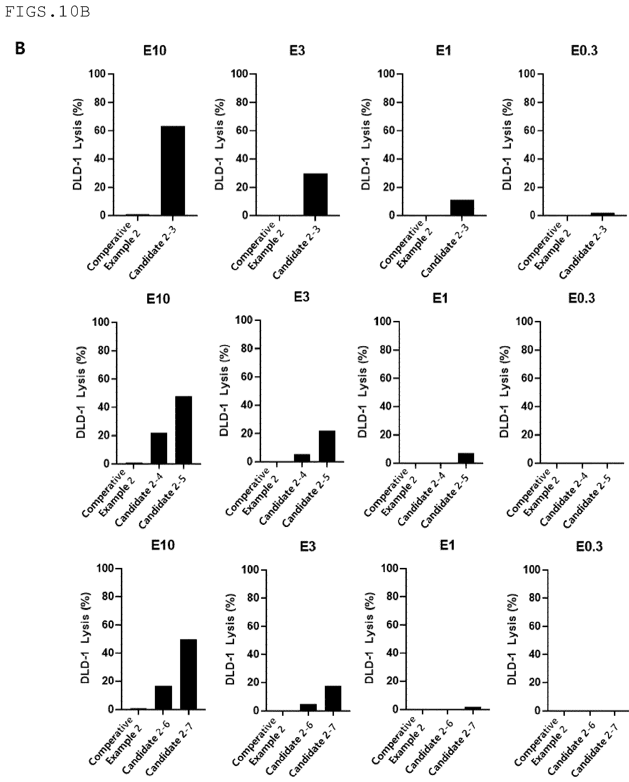

Consequently, as shown in FIGS. 10A and 10B, candidates 2-3 to 2-7 were confirmed to restore the ability of NK cells to kill cancer cells after thawing, whereas Comparative Example 2 expressed the cell surface markers or cytotoxicity markers at the same level as candidates 2-3 to 2-7 but was confirmed not to restore the ability to kill cancer cells in practice. Therefore, it was concluded that the PEG of the composition for cryopreservation included in candidates 2-3 to 2-7 had an effect of restoring the ability of NK cells to kill cancer cells after thawing.

### Industrial Applicability

According to the present invention, a composition for NK cell cryopreservation is capable of exhibiting cell surface marker expression and NK cell degranulation ability at at least the same level as formulations using currently commercially available cryopreservation media, even in low-concentration DMSO not including HEPES (hydroxyethyl piperazine ethane sulfonic acid) or HES (hydroxyethyl starch), and also of showing a high cell yield and vastly superior ability of NK cells to kill cancer cells. In particular, since a conventional cryoprotectant showing various side effects due to nonspecific toxicity is not included or is comprised at a low concentration and is replaced with a nontoxic and biocompatible cryoprotectant, the composition can be administered to a patient immediately after thawing without additional culture or replacement of the solution with an injectable solution, so it can be used for a pharmaceutical formulation. Therefore, the composition is very useful for long-term storage and clinical application of NK-cell-based immune cell therapeutic agents for therapy for very limited indications.

Although specific embodiments of the present invention have been disclosed in detail above, it will be obvious to those skilled in the art that the description is merely of preferable exemplary embodiments, and is not to be construed as limiting the scope of the present invention. Therefore, the substantial scope of the present invention will be defined by the appended claims and equivalents thereof.

## Claims

1. A composition for NK (natural killer) cell cryopreservation, comprising:
(i) a base solution;
(ii) serum albumin;
(iii) DMSO (dimethyl sulfoxide); and
(iv) at least one cryoprotectant selected from the group consisting of glucose, polyethylene glycol, and trehalose.

2. The composition according to claim 1, wherein the base solution comprises at least one selected from among sodium, chloride, potassium, magnesium, acetate, and gluconate.

3. The composition according to claim 1, wherein a pH of the base solution is 4.0 to 8.0.

4. The composition according to claim 1, wherein an osmolality of the base solution is 200 mOsm/L to 400 mOsm/L.

5. The composition according to claim 1, wherein the DMSO is comprised at a final concentration of 0.001% (v/v) to 10% (v/v).

6. The composition according to claim 1, not comprising HEPES (hydroxyethyl piperazine ethane sulfonic acid) or HES (hydroxyethyl starch).

7. The composition according to claim 1, wherein the serum albumin is comprised at a final concentration of 1% (w/v) to 10% (w/v).

8. The composition according to claim 1, wherein the cryoprotectant is any one selected from the group consisting of glucose, polyethylene glycol, and trehalose.

9. The composition according to claim 8, wherein the cryoprotectant is glucose, and the glucose is comprised at a final concentration of 0.5% (w/v) to 5% (w/v).

10. The composition according to claim 8, wherein the cryoprotectant is polyethylene glycol, and the polyethylene glycol has an average molecular weight of 400 g/mol to 20000 g/mol.

11. The composition according to claim 8, wherein the cryoprotectant is polyethylene glycol, and the polyethylene glycol is comprised at a final concentration of 0.045% (w/v) to 2.5% (w/v).

12. The composition according to claim 8, wherein the cryoprotectant is trehalose, and the trehalose is comprised at a final concentration of 5% (w/v) to 10% (w/v).

13. An NK cell cryopreservation formulation, comprising:
NK cells; and
the composition according to any one of claims 1 to 12.

14. A method of cryopreserving NK cells, comprising:
preparing an NK cell cryopreservation formulation by suspending NK cells in the composition according to any one of claims 1 to 12; and
freezing the NK cell cryopreservation formulation.

15. An immune cell therapeutic agent, comprising:
NK cells; and
the composition according to any one of claims 1 to 12.

16. A pharmaceutical composition for preventing or treating cancer, an immune disease, or an infectious disease, comprising:
NK cells; and
the composition according to any one of claims 1 to 12.

17. A method of preventing or treating cancer, an immune disease, or an infectious disease using the NK cell cryopreservation formulation according to claim 13.
